(19) Europäisches Patentamt
European Patent Office
Office européen des brevets

(11) **EP 3 569 147 B1**

(12) **EUROPÄISCHE PATENTSCHRIFT**

(45) Veröffentlichungstag und Bekanntmachung des Hinweises auf die Patenterteilung:
**21.07.2021   Patentblatt 2021/29**

(51) Int Cl.:
***A61B 6/00*** *(2006.01)*

(21) Anmeldenummer: **18172655.5**

(22) Anmeldetag: **16.05.2018**

(54) **VERFAHREN UND VORRICHTUNG ZUM BESTIMMEN EINER GEOMETRIEKALIB-RIERUNG FÜR EINE BILDGEBENDE VORRICHTUNG SOWIE VERFAHREN ZUM BESTIMMEN VON ZUORDNUNGSDATEN FÜR DIE GEOMETRIEKALIBRIERUNG**

METHOD AND DEVICE FOR DETERMINING A GEOMETRIC CALIBRATION FOR AN IMAGING DEVICE AND METHOD FOR DETERMINING ALLOCATION DATA FOR GEOMETRIC CALIBRATION

PROCÉDÉ ET APPAREIL DE DÉTERMINATION D'UN ÉTALONNAGE GÉOMÉTRIQUE POUR UN DISPOSITIF D'IMAGERIE AINSI QUE PROCÉDÉ DE DÉTERMINATION DE DONNÉES D'ATTRIBUTION POUR ÉTALONNAGE GÉOMÉTRIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**20.11.2019   Patentblatt 2019/47**

(73) Patentinhaber: **Siemens Healthcare GmbH**
**91052 Erlangen (DE)**

(72) Erfinder:
- **Aichert, André**
  **91052 Erlangen (DE)**
- **Maier, Andreas**
  **91054 Erlangen (DE)**
- **Würfl, Tobias**
  **91052 Erlangen (DE)**

(56) Entgegenhaltungen:
**WO-A2-01/87136          US-A1- 2017 074 808**
**US-A1- 2017 103 505**

**Beschreibung**

Gebiet der Erfindung

Die Erfindung liegt auf dem Gebiet der Transmissionsbildgebung und betrifft insbesondere ein Verfahren zum Bestimmen einer Geometriekalibrierung für eine bildgebende Vorrichtung, eine Vorrichtung zum Bestimmen einer Geometriekalibrierung für eine bildgebende Vorrichtung, ein 3-D Kalibrierphantom zum Bestimmen einer Geometriekalibrierung und zum Bestimmen von Zuordnungsdaten für eine Geometriekalibrierung sowie ein Verfahren zum Bestimmen von Zuordnungsdaten zur Geometriekalibrierung einer bildgebenden Vorrichtung.

Hintergrund

In der Transmissionsbildgebung wird zum Erstellen eines Transmissionsbilds Strahlung durch ein zu untersuchendes Objekt geleitet und trifft nach Durchgang durch das zu untersuchende Objekt auf einem 2-D Detektor auf. Eine solche Transmissionsbildgebung ist besonders aus der Computertomographie bekannt. Dabei ist die bildgebende Vorrichtung ein Computertomograph, welcher als Strahlungsquelle üblicherweise eine Röntgenquelle aufweist und mittels seines 2-D Detektors erfasst, inwiefern die (Röntgen-) Strahlung durch das zu untersuchende Objekt verändert, insbesondere absorbiert worden ist. Bei Computertomographiesystemen besteht ein Trend hin zu offeneren und damit insbesondere flexibleren Systemen. So bewegen sich etwa anders als bei konventionellen Computertomographiesystemen aktuelle C-Bögen mit einem Roboterarm, an dem die Strahlenquelle und der 2-D Detektor angebracht ist, um das zu untersuchende Objekt. Auf diese Weise wird keine starre Röhre benötigt und daher benötigen solche Systeme üblicherweise weniger Platz und können flexibler eingesetzt werden - was etwa im interventionellen Einsatz oder bei der Materialprüfung verschiedenster Teile in der Industrie besonders relevant ist. Allerdings ist die Geometrie solcher Systeme üblicherweise - etwa aufgrund mechanischer Beschränkungen - kein perfekter Kreisbogen oder keine perfekte Spirale und/oder weniger genau bekannt.

Bei bildgebenden Systemen deren exakte Geometrie und/oder Abbildungseigenschaften - etwa aufgrund von mechanischen Einschränkungen und/oder ihrer Betriebsweise - nicht exakt bekannt sind, welche jedoch die zu untersuchenden Objekte in reproduzierbarer Weise abbilden, lässt sich eine geometrische Kalibrierung durchführen. So können etwa die Strahlungsquelle und der 2-D Detektor eines Computertomographiesystems mit C-Bogen in reproduzierbarer Weise um das zu untersuchende Objekt bewegt werden und so reproduzierbar Abbildungen, d.h. insbesondere mit reproduzierbaren Fehlern, erstellt werden. Durch eine geometrische Kalibrierung lassen sich diese reproduzierbaren Fehler ausgleichen. Die für eine solche geometrische Kalibrierung erforderlichen Daten für eine Geometriekalibrierung - kurz Geometriekalibrierung - werden üblicherweise durch eine dedizierte Aufnahme eines bekannten und exakt gefertigten Testobjekts (eines sogenannten 3-D Kalibrierphantoms) für jedes Einzelbild - also je Ansicht des 3-D Kalibrierphantoms und dessen jeweiligen 2-D Transmissionsbilds - bestimmt. Typischerweise weist ein solches 3-D Kalibrierphantom mehrere exakt positionierte Metallkugeln auf, wie etwa das PDS2 aus N. K. Strobel et al, Improving 3-D Image Quality of X-Ray C-Arm Imaging Systems by Using Properply Designed Pose Determination Systems for Calibrating the Projection Geometry, Proceedings of SPIE.

Es ist zu beachten, dass in einem 2-D Transmissionsbild die bei einem 3-D Kalibrierphantom üblicherweise verwendeten Metallkugeln alle gleich ausschauen, also insbesondere bei der Computertomographie nur ihre jeweiligen Schatten in der Röntgenstrahlung sichtbar sind, so dass diese untereinander nicht unterschieden werden können. Bei den üblicherweise verwendeten Algorithmen zur geometrischen Kalibrierung ist man auf einige 3-D Kalibrierphantome sowie dafür speziell angepasste Trajektorien - d. h. insbesondere Bahnen für bestimmte Ansichten und damit 2-D Transmissionsbilder des jeweiligen 3-D Kalibrierphantoms aus verschiedenen Positionen des 2-D Detektors und/oder der Strahlenquelle - beschränkt. Auch besteht eine Beschränkung auf jene räumlichen Ausdehnungen, für welche solche Kalibrierphantome vorhanden sind.

WO 01/87136 A2 beschreibt ein System, bei dem Markierungen verwendet werden, um die Projektionsgeometrie zu bestimmen.

Die US 2017/0074808 A1 beschreibt eine Testgeometrie zur Kalibrierung von Röntgeneinrichtungen.

Zusammenfassung der Erfindung

Es besteht Bedarf, die geometrische Kalibrierung zu verbessern und dabei insbesondere die Herstellung eines 3-D Kalibrierphantoms zu erleichtern und/oder flexibler zu gestalten und/oder die Beschränkungen bei der geometrischen Kalibrierung und deren Durchführung zu reduzieren.

Die Erfindung löst die Aufgabe jeweils durch ein Verfahren zum Bestimmen einer Geometriekalibrierung für eine bildgebende Vorrichtung gemäß der Lehre des Hauptanspruchs. Vorteilhafte Ausführungsformen, Weiterbildungen und Varianten der vorliegenden Erfindung sind insbesondere Gegenstand der Unteransprüche.

Ein erster Aspekt der Erfindung betrifft ein Verfahren zum Bestimmen einer Geometriekalibrierung für eine bildgebende Vorrichtung gemäß Anspruch 1, bei welcher in einer Transmissionsbildgebung eine Strahlung nach Durchgang durch ein 3-D Kalibrierphantom zur Erstellung eines 2-D Transmissionsbilds des 3-D Kalibrierphantoms auf einem 2-D Detektor auftrifft. Das 3-D Kalibrierphantom weist wenigstens ein Kalibrierobjekt mit einer vorbestimmten Anzahl an physischen Kalibrierelementen auf, welche derart räumlich angeordnet sind, dass ein auf der räumlichen Anordnung basierender Deskriptor unter der Transmissionsbildgebung projektiv invariant ist. Dabei kann in einigen Varianten der Deskriptor zudem darauf beruhen, dass eines oder mehrere der physischen Kalibrierelemente so ausgebildet sind, dass diese sich - auch im 2-D Transmissionsbild - von den übrigen physischen Kalibrierelementen unterscheiden. Das Verfahren weist folgendes auf. Bei dem Verfahren wird das 2-D Transmissionsbild, in dem das wenigstens eine Kalibrierobjekt des 3-D Kalibrierphantoms dargestellt ist, bereitgestellt. Zudem werden bei dem Verfahren die in dem 2-D Transmissionsbild dargestellten physischen Kalibrierelemente zur Bestimmung von im 2-D Transmissionsbild erkannten Kalibrierelementen und ihrer 2-D Anordnung erkannt. Beim Verfahren werden Untermengen der erkannten Kalibrierelemente bestimmt, deren Anzahl an Kalibrierelementen jeweils der vorbestimmten Anzahl an physischen Kalibrierelementen des wenigstens einen Kalibrierobjekts entspricht. Je Untermenge wird jeweils der Deskriptor bezüglich der Kalibrierelemente berechnet. Schließlich werden die Zuordnungsdaten bestimmt, indem die erkannten Kalibrierelemente jeder Untermenge den physischen Kalibrierelementen des wenigstens einen Kalibrierobjekts zugeordnet werden, für welche der berechnete Deskriptor und der Deskriptor bezüglich der physischen Kalibrierelemente am wenigsten voneinander abweichen.

Im Sinne der Erfindung ist unter einem "Kalibrierelement" zumindest ein Teil eines Kalibrierobjekts und/oder eines 3-D Kalibrierphantoms zu verstehen, wobei der Teil so ausgebildet ist und insbesondere ein solches Material aufweist, dass die Strahlung durch das Kalibrierelement beeinflusst wird und im 2-D Transmissionsbild ein Bild des Kalibrierelements entsteht. Bei einer Röntgenstrahlung als Strahlung - etwa bei der Computertomographie - kann das Kalibrierelement aus einem Metall gefertigt sein. Auch kann das Kalibrierelement als eine Kugel mit einer bestimmten räumlichen Ausdehnung geformt sein.

Im Sinne der Erfindung ist unter einem "Deskriptor" für ein Kalibrierobjekt zumindest eine Information zu verstehen, welche dieses Kalibrierobjekt kennzeichnet. Dabei können diese Informationen Eigenschaften der räumlichen Anordnung der Kalibrierelemente dieses Kalibrierobjekts umfassen. Abhängig vom Grad der Kennzeichnungskraft der Information - also des Deskriptors - für dieses Kalibrierobjekt spricht man auch von einem schwachen oder einem starken Deskriptor. Dabei kann ein schwacher Deskriptor gleiche Informationen für verschiedene Kalibrierobjekte aufweisen, wodurch keine eindeutige Identifizierung des Kalibrierobjekts ermöglicht wird. Umgekehrt kann ein starker Deskriptor so ausgebildet sein, d. h. die Informationen so gewählt sein, dass - insbesondere bei einer beschränkten Anzahl und Art von Kalibrierobjekten - die Kalibrierobjekte jeweils über diesen starken Deskriptor (eindeutig) identifizierbar sind.

Ein Vorteil des Bestimmens der Zuordnungsdaten kann insbesondere darin liegen, dass die im 2-D Transmissionsbild erkannten Kalibrierelemente den physischen Kalibrierelementen des wenigstens einen Kalibrierobjekts des 3-D Kalibrierphantoms zugeordnet werden können, wodurch für die geometrische Kalibrierung - also insbesondere zur Bestimmung einer Geometriekalibrierung - Algorithmen aus der Computer Vision einsetzen lassen. Derartige Algorithmen aus der Computer Vision gehen davon aus, dass sich die abgebildeten Objekte in einem Bild ihren physischen Objekten zuordnen lassen. Da aber üblicherweise die in der Transmissionsbildgebung verwendeten Kalibrierelemente alle zumindest im Wesentlichen gleiche Abbilder aufweisen, also sich insbesondere im 2-D Transmissionsbild zumindest im Wesentlichen gleichen, können die Algorithmen aus der Computer Vision nicht unmittelbar angewandt werden. Vielmehr ermöglicht erst das Bestimmen der Zuordnungsdaten ein Übertragen dieser Algorithmen auf die geometrische Kalibrierung bei der Transmissionsbildgebung. Anhand des Deskriptor, welcher unter der Transmissionsbildgebung projektiv invariant ist, lassen sich die Kalibrierobjekte bzw. das wenigstens eine Kalibrierobjekt des 3-D Kalibrierphantoms - zumindest statistisch - identifizieren unabhängig von der jeweiligen Ansicht, d. h. insbesondere von der Projektion des wenigstens einen Kalibrierobjekts auf das 2-D Transmissionsbild. Somit wird vorteilhaft eine Zuordnung des wenigstens einen Kalibrierobjekts und seinem Abbild im 2-D Transmissionsbild ermöglicht, womit insbesondere auch die Kalibrierelemente des wenigstens einen Kalibrierobjekts ihren jeweiligen Abbildern zugeordnet werden können. Dadurch ist man insbesondere nicht mehr auf wenige exakt gefertigte Kalibrierphantome und entsprechende Trajektorien, welche eine Zuordnung der Elemente des Kalibrierphantoms zu den jeweiligen Abbildungen davon unter den Ansichten der jeweiligen Trajektorien ermöglichen, beschränkt. Ohne diese Beschränkung kann ein 3-D Kalibrierphantom insbesondere an die zu untersuchenden Objekte angepasst werden - etwa durch Anordnen mehrerer Kalibrierobjekte verteilt im Raum und/oder nahe an den zu erwartenden Bildrändern der zu untersuchenden Objekte - wodurch eine für diese zu untersuchenden Objekte exaktere Kalibrierung ermöglicht wird.

Bei einigen Ausführungsformen weist das 3-D Kalibrierphantom eine Vielzahl an Kalibrierobjekten auf, wobei das wenigstens eine Kalibrierobjekt und ein weiteres Kalibrierobjekt jeweils Teil der Vielzahl an Kalibrierobjekten sind. Zudem weisen die Kalibrierobjekte der Vielzahl an Kalibrierobjekten jeweils eine vorbestimmte Anzahl an physischen Kalibrierelementen auf, welche derart räumlich angeordnet sind, dass ein auf der räumlichen Anordnung basierender Deskriptor unter der Transmissionsbildgebung projektiv invariant ist, wobei dieser Deskriptor ein starker Deskriptor ist. Ein Vorteil eines solchen starken Deskriptors kann insbesondere darin liegen, dass sich die Kalibrierobjekte der Vielzahl an Kalibrierobjekten auf Basis des starken Deskriptors - welcher also ein starker Deskriptor für diese Vielzahl ist - (insbesondere eindeutig) identifizieren lassen.

Bei einigen Ausführungsformen, für welche das 3-D Kalibrierphantom eine Vielzahl an Kalibrierobjekten auf, wird das Verfahren für die physischen Kalibrierelemente der Kalibrierobjekte der Vielzahl an Kalibrierobjekten entsprechend ausgeführt. Zudem weist das Verfahren ein Identifizieren der einzelnen Kalibrierobjekte der Vielzahl an Kalibrierobjekten basierend auf dem starken Deskriptor auf.

Bei einigen Ausführungsformen des Verfahrens, für welches das wenigstens eine Kalibrierobjekt vier physische Kalibrierelemente aufweist, die zumindest im Wesentlichen entlang einer ersten Geraden räumlich angeordnet sind, kann der Deskriptor eine Anordnung der erkannten Kalibrierelemente zumindest im Wesentlichen entlang einer 2-D Geraden sowie ein Doppelverhältnis für die Abstände der erkannten Kalibrierelemente untereinander aufweisen. Im Verfahren werden beim Bestimmen der Untermengen jene bestimmt, bei welchen die vier erkannten Kalibrierelemente der jeweiligen Untermenge zumindest im Wesentlichen entlang einer 2-D Geraden im 2-D Transmissionsbild angeordnet sind. Zudem wird jeweils der Deskriptor je Untermenge jeweils mittels des Doppelverhältnisses für die Abstände der vier erkannten Kalibrierelemente untereinander berechnet. Ein Vorteil dieses Deskriptors kann insbesondere darin liegen, dass er zwei Informationen - nämlich dass die Kalibrierelemente entlang einer 2-D Geraden im Transmissionsbild bzw. entlang einer 3-D Geraden bei dem 3-D Kalibrierphantom angeordnet sind, und das Doppelverhältnis - aufweist, welche jeweils projektiv invariant sind und welche zunächst anhand der Anordnung entlang der Geraden eine effiziente Aufteilung in Untermengen ermöglichen und anschließend - sofern die Kalibrierobjekte des 3-D Kalibrierphantoms so gewählt sind, dass sich die Doppelverhältnisse der Kalibrierobjekte unterscheiden - eine Unterscheidung und damit Identifizierung der Kalibrierobjekte und ihrer Kalibrierelemente und somit insbesondere eine Zuordnung der physischen Kalibrierelemente zu ihren Abbildern im 2-D Transmissionsbild ermöglichen. Auch lassen sich die Doppelverhältnisses so wählen, dass diese für die unterschiedlichen Kalibrierobjekte soweit unterschiedlich sind, dass - zumindest bis zu einem gewissen Grad - Abweichungen bei der Fertigung der Kalibrierobjekte, d. h. insbesondere bezüglich der Abstände ihrer Kalibrierelemente, tolerierbar sind, da auch bei einer Abweichung von den idealen Abständen für dieses Kalibrierobjekt und seines Doppelverhältnisses das konkrete Doppelverhältnis von dem idealen Doppelverhältnis weniger abweicht als von den Doppelverhältnissen der anderen Kalibrierobjekte.

In einigen vorteilhaften Varianten ist das Doppelverhältnis ein starker Deskriptor.

Bei einigen Ausführungsformen des Verfahrens, für welches das wenigstens eine Kalibrierobjekt oder ein weiteres Kalibrierobjekt sieben physische Kalibrierelemente aufweist, von denen eines auf einer ersten Geraden und auf einer zweiten Geraden, welche nicht parallel sind, liegt und von denen jeweils drei weitere zumindest im Wesentlichen entlang der ersten bzw. der zweiten Geraden räumlich angeordnet sind, kann der Deskriptor zum einen eine Anordnung von vier erkannten Kalibrierelementen zumindest im Wesentlichen entlang einer ersten 2-D Geraden sowie ein erstes Doppelverhältnis für die Abstände dieser vier Kalibrierelemente untereinander und zum anderen eine Anordnung von vier erkannten Kalibrierelementen entlang einer zweiten 2-D Geraden, welche die erste 2-D Gerade in einem dieser Kalibrierelemente schneidet, sowie ein zweites Doppelverhältnis für die Abstände dieser Kalibrierelemente untereinander aufweisen. Zudem werden beim Bestimmen der Untermengen jene Untermengen bestimmt, welche sieben erkannte Kalibrierelemente aufweisen, von denen vier entlang einer ersten 2-D Geraden und vier entlang einer zweiten 3-D Geraden im 2-D Transmissionsbild angeordnet sind. Außerdem wird im Verfahren jeweils der Deskriptor je Untermenge des Weiteren mittels des ersten Doppelverhältnisses für die Abstände der vier erkannten Kalibrierelemente entlang der ersten 2-D Geraden und mittels des Doppelverhältnisses für die Abstände der vier Kalibrierelemente entlang der zweiten 2-D Geraden berechnet. Insbesondere durch das Kalibrierelement im Schnittpunkt der beiden Geraden lässt sich eine Reihenfolge der Kalibrierelemente vorteilhaft festlegen.

In einigen vorteilhaften Varianten mit einem Deskriptor, der mehrere Informationen aufweist, kann ein erster Teil dieser Informationen implizit gegeben sein. Dabei kann in einigen vorteilhaften Varianten ein zweiter Teil der Informationen des Deskriptors vom Vorliegen des ersten Teils abhängen bzw. erforderlich sein, um den zweiten Teil zu bestimmen. So ist das Doppelverhältnis insbesondere dann definiert, wenn die Kalibrierelemente entlang einer Geraden angeordnet sind. Somit wird in einigen vorteilhaften Varianten das Doppelverhältnis abhängig davon als Deskriptor bestimmt, dass die Kalibrierelemente entlang einer Geraden angeordnet sind.

In einigen Ausführungsformen lässt sich das Doppelverhältnis im dreidimensionalen, d. h. insbesondere für das 3-D Kalibrierphantom und für seine physischen Kalibrierelemente, folgendermaßen bestimmen:

Gegeben seien die Vektoren der vier entlang einer Geraden angeordneten Kalibrierelemente: **a**, **b**, **c** und **d**.

Damit ergeben sich als Einheitsvektor entlang der Geraden

$$\mathbf{i} = \frac{\mathbf{a} - \mathbf{b}}{||\mathbf{a} - \mathbf{b}||} \in \mathbb{R}^3$$

und als (relative) Abstände entlang der Geraden

$$a \stackrel{\text{def}}{=} 1 \in R$$

$$b \stackrel{\text{def}}{=} 0 \in R$$

$$c \stackrel{\text{def}}{=} \mathbf{i}^{\mathrm{T}}(\mathbf{c} - \mathbf{b}) \in R$$

$$d \stackrel{\text{def}}{=} \mathbf{i}^{\mathrm{T}}(\mathbf{d} - \mathbf{b}) \in R$$

und als Doppelverhältnis

$$\lambda = cr\,(a, b; c, d) \stackrel{\text{def}}{=} \frac{(a - c) \cdot (b - d)}{(a - d) \cdot (b - c)} \in R$$

Im Zweidimensionalen kann das Doppelverhältnis entsprechend bestimmt werden. Dabei entspricht der im Dreidimensionalen bestimmte Wert des Doppelverhältnisses für die physischen Kalibrierelemente dem im Zweidimensionalen bestimmten Wert für die entsprechenden im 2-D Transmissionsbild erkannten Kalibrierelemente, da das Doppelverhältnis projektiv invariant ist.

In einigen Ausführungsformen kann der Wert des Doppelverhältnisses von der Reihenfolge der Kalibrierelemente abhängen - also insbesondere davon, welchem der Kalibrierelemente man den Vektor **a** zuordnet usf. Somit können sich bei vier Kalibrierelementen sechs Permutationen und entsprechend sechs Werte, nämlich

$$\lambda, \frac{1}{\lambda}, 1 - \lambda, \frac{1}{1 - \lambda}, \frac{\lambda - 1}{\lambda}, \frac{\lambda}{\lambda - 1}$$

ergeben. In einigen vorteilhaften Varianten, werden zum Bestimmen der Zuordnungsdaten alle sechs Werte berechnet.

In einigen Ausführungsformen kann das Verfahren des Weiteren ein Festlegen einer Reihenfolge der Kalibrierelemente für jede Untermenge mittels eines vorbestimmten Kriteriums für die Reihenfolge aufweisen. Auf diese vorteilhafte Weise lassen sich die erkannten Kalibrierelemente der Untermenge, welche dem wenigstens einen Kalibrierobjekts zugeordnet ist, jeweils ihrem jeweiligen physischen Kalibrierelement eindeutig zuordnen.

In einigen vorteilhaften Varianten kann das vorbestimmte Kriterium für die Reihenfolge ein Schnittpunkt zweier Geraden sein, entlang welcher die Kalibrierelemente des wenigstens einen Kalibrierobjekts angeordnet sind.

Bei einigen Ausführungsformen, bei welchen im Verfahren eine Reihenfolge der Kalibrierelemente festgelegt wird, kann der Deskriptor diese Reihenfolge aufweisen. Auf diese vorteilhafte Weise weist der Deskriptor eine weitere Information auf, welche eine Unterscheidung zwischen zwei Kalibrierobjekten ermöglicht, auch wenn diese sich bis auf die Reihenfolge - zumindest bezüglich der Informationen des Deskriptors - zumindest im Wesentlichen gleichen.

Alternativ oder zusätzlich kann in einigen Ausführungsformen, in welchen eine Reihenfolge der Kalibrierelemente festgelegt wird, diese Reihenfolge dazu verwendet werden, eine weitere Information des Deskriptor unabhängig von einer etwaigen Permutation in der Reihenfolge bzw. Anordnung der Kalibrierelemente des jeweiligen Kalibrierobjekts zu machen. So ergeben sich etwa bei dem Doppelverhältnis für ein Kalibrierobjekt abhängig davon unterschiedliche Werte, welches der Kalibrierelemente des Kalibrierobjekts einer ersten, einer zweiten, einer dritten oder einer vierten Position zugeordnet wird, wodurch sich unterschiedliche Abstände ergeben. Dadurch dass eine bestimmte Reihenfolge festgelegt wird, lässt sich eine bestimmte Reihenfolge und damit ein bestimmter Wert des Doppelverhältnisses für eine bestimmte Permutation der Kalibrierelemente bzw. ihrer Positionen festlegen, wodurch der Wert dieses Doppelverhältnisses eindeutig für das Kalibrierobjekt wird.

Bei einigen Ausführungsformen kann das wenigstens eine Kalibrierobjekt oder ein weiteres Kalibrierobjekt wenigstens ein unterschiedliches Kalibrierobjekt, das sich gegenüber den übrigen Kalibrierobjekten bezüglich eines Unterscheidungskriteriums unterscheidet, aufweisen. In einigen vorteilhaften Varianten kann sich das unterschiedliche Kalibrierelement darin unterscheiden, dass dieses eine größere oder eine kleinere räumliche Ausdehnung aufweist. Auch kann in einigen vorteilhaften Varianten das Unterscheidungskriterium darin bestehen, dass das unterschiedliche Kalibrierelement die Strahlung anders streut oder absorbiert. Auf diese vorteilhafte Weise lässt sich das unterschiedliche Kalibrierelement von den übrigen Kalibrierelementen - d. h. zumindest von den übrigen Kalibrierelementen, welche räumlich in der Nähe des unterschiedlichen Kalibrierelements angeordnet sind und damit ähnlich, insbesondere pseudo-affin, abgebildet werden - im 2-D Transmissionsbild anhand des Unterscheidungskriteriums unterscheiden.

Bei einigen Ausführungsformen, bei welchen im Verfahren eine Reihenfolge der Kalibrierelemente festgelegt wird und bei welchen das wenigstens eine Kalibrierobjekt oder ein weiteres Kalibrierobjekt wenigstens ein unterschiedliches Kalibrierelement aufweist, weist das Verfahren des Weiteren folgendes auf. Je erkanntem Kalibrierelement wird jeweils das Unterscheidungskriterium auf dem 2-D Transmissionsbild bestimmt. Je Untermenge wird bestimmt, ob und welche der erkannten Kalibrierelemente der jeweiligen Untermenge sich relativ zu den übrigen Kalibrierelementen durch ihr jeweiliges Unterscheidungskriterium unterscheiden. Dabei wird insbesondere davon ausgegangen, dass die Kalibrierelemente einer Untermenge auch räumlich zumindest im Wesentlichen benachbart zueinander angeordnet sind. Dabei kann in einigen vorteilhaften Varianten das vorbestimmte Kriterium für die Reihenfolge auf dem Unterscheidungskriterium basieren. Alternativ oder zusätzlich kann in einigen vorteilhaften Varianten der Deskriptor eine Position des wenigstens einen unterschiedlichen Kalibrierelements innerhalb der Reihenfolge der Kalibrierelemente aufweisen. Auf diese vorteilhafte Weise lässt sich die Reihenfolge der Kalibrierelemente anhand des wenigstens einen unterschiedlichen Kalibrierelements festlegen. Auch lassen sich Kalibrierobjekte mit ansonsten zumindest im Wesentlichen gleichen Eigenschaften anhand der Position des wenigstens einen unterschiedlichen Kalibrierelements unterscheiden.

In einigen Ausführungsformen weist das 3-D Kalibrierphantom ein weiteres Kalibrierobjekt mit einer vorbestimmten Anzahl an physischen Kalibrierelementen auf, welche entsprechend den physischen Kalibrierelementen des wenigstens einen Kalibrierobjekts derart räumlich angeordnet und/oder ausgebildet sind, dass sich der Deskriptor bezüglich des weiteren Kalibrierobjekts von dem Deskriptor bezüglich des wenigstens einen Kalibrierobjekts unterscheidet.

In einigen Ausführungsformen das Verfahrens, für welche das 3-D Kalibrierphantom ein weiteres Kalibrierobjekt aufweist, kann das Verfahren für die physischen Kalibrierelemente des weiteren Kalibrierobjekts entsprechend ausgeführt werden. Dabei werden, sofern die vorbestimmte Anzahl an physischen Kalibrierelementen für das wenigstens eine Kalibrierobjekt und das weitere Kalibrierobjekt gleich sind, die erkannten Kalibrierelemente jener Untermenge entweder den physischen Kalibrierelementen des wenigstens einen Kalibrierobjekts oder den physischen Kalibrierelementen des anderen Kalibrierobjekts abhängig davon zugeordnet, ob der berechnete Deskriptor für diese Untermenge von dem Deskriptor des wenigstens einen Kalibrierobjekts oder von dem Deskriptor des weiteren Kalibrierobjekts weniger abweicht. Auf diese vorteilhafte Weise kann das 3-D Kalibrierphantom weitere Kalibrierelemente und/oder weitere Kalibrierobjekte aufweisen, so dass sich insgesamt eine größere Anzahl an Kalibrierelementen ergibt, wodurch insbesondere die Güte der Geometriekalibrierung erhöht werden kann.

In einigen Ausführungsformen weist das Verfahren des Weiteren ein Bereitstellen der Zuordnungsdaten auf. In einigen vorteilhaften Varianten können die Zuordnungsdaten dabei mittels einer Datenschnittstelle ausgegeben werden. Auf diese vorteilhafte Weise lassen sich die Zuordnungsdaten zum Bestimmen der Geometriekalibrierung verwenden und/oder vor dem Bestimmen der Geometriekalibrierung überprüfen.

In einigen Ausführungsformen weist das Verfahren des Weiteren ein markerbasiertes 3D/3D Registrieren auf Basis der Zuordnungsdaten auf. In einigen vorteilhaften Varianten werden alternativ oder zusätzlich zur Geometriekalibrierung eines oder mehrere Transmissionsbilder, insbesondere eines Patienten oder eines zu untersuchenden Objekts, mittels des 3-D Kalibrierphantoms gekennzeichnet. Insbesondere durch die projektive Invarianz des Deskriptors/der Deskriptoren lässt sich (für Transmissionsbilder aus verschiedenen Ansichten) eine eindeutige Zuordnung anhand einer 3D Registrierung durch das 3D Registrieren ermöglichen. Weiter lassen sich in einigen vorteilhaften Varianten die Kalibrierobjekte oder -elemente flexibel anordnen und so flexibel unterschiedliche 3D Registrierungen erstellen.

Auch lassen sich in einigen Ausführungsformen zu untersuchende Objekte oder Teile davon anhand des 3D Kalibierphantoms kennzeichnen, insbesondere entsprechend einer 3D Registrierung. Auf diese vorteilhafte Weise lassen sich die Positionen von Objekten und besonders vorteilhaft von bestimmten Teilen von Objekten kennzeichnen, wodurch insbesondere ein Auffinden von Teilen eines zu untersuchenden Objekts im Transmissionsbild und/oder ein Bestimmen ihrer räumlichen Positionen ermöglicht wird.

Im Sinne der Erfindung umfasst die Transmissionsbildgebung insbesondere die Fluoroskopie.

Bei einigen Ausführungsformen, bei welchen eine Fluoroskopie durchgeführt wird, wird ein Patient oder ein zu untersuchendes Objekt oder Bereiche oder Teile davon mittels des 3D Kalibierphantoms, etwa vorteilhaft durch 3D Registrieren, gekennzeichnet.

In einigen Ausführungsformen kann das Verfahren des Weiteren ein Berechnen jeweils eines ersten Fehlerwertes je Untermenge, welcher die Abweichung zwischen dem für diese Untermenge berechneten Deskriptor und dem Deskriptor des wenigstens einen Kalibrierobjekts oder eines weiteren Kalibrierobjekts kennzeichnet, aufweisen. Zudem werden die erkannten Kalibrierelemente einer der Untermengen den physischen Kalibrierelementen des wenigstens einen bzw. des weiteren Kalibrierobjekts abhängig davon zugeordnet, ob der jeweilige erste Fehlerwert geringer als ein erster Grenzwert ist.

In einigen Ausführungsformen wird das Verfahren mit unterschiedlich bestimmten Untermengen der erkannten Kalibrierelemente wiederholt ausgeführt. Insbesondere kann in einigen vorteilhaften Varianten ein so genannter RANSAC-Algorithmus ausgeführt werden, so dass das Verfahren iterativ ausgeführt wird und die Zuordnung der erkannten Kalibrierelemente zu den physischen Kalibrierelementen adaptiv erfolgt und dabei insbesondere jeweils ein erster Fehlerwert und/oder ein Gesamtfehlerwert, welcher die ersten Fehlerwerte umfasst, minimiert wird. Ein Vorteil der wiederholten Ausführung kann insbesondere darin liegen, dass Fehler bei der Zuordnung reduziert werden können, wodurch sich insbesondere die Güte einer anschließenden Geometriekalibrierung verbessern lässt. Ein Vorteil der adaptiven Zuordnung durch Anwenden des RANSAC-Algorithmus kann insbesondere darin liegen, dass RANSAC ein statistischer Algorithmus ist, wodurch sich auch bei Fehlern in den Zuordnungsdaten und/oder auch bei schwachen Deskriptoren zumindest für eine echte Teilmenge der erkannten Kalibierelemente eine korrekte Zuordnung - also insbesondere eine korrekte minimale Zuordnung - bestimmen lässt. Somit kann das Anwenden des RANSAC-Algorithmus insbesondere die Robustheit des Verfahrens erhöhen. Auch kann in einigen vorteilhaften Varianten die Geometriekalibrierung bereits basierend auf einer solchen minimalen Zuordnung durchgeführt werden.

In einigen Ausführungsformen weist das 3-D Kalibrierphantom ein weiteres Kalibrierobjekt mit einer vorbestimmten Anzahl an physischen Kalibrierelementen auf, welche entsprechend den physischen Kalibrierelementen des wenigstens einen Kalibrierobjekts derart räumlich angeordnet und/oder entsprechend den physischen Kalibrierelementen des wenigstens einen Kalibrierobjekts derart ausgebildet sind, dass sich der Deskriptor bezüglich des weiteren Kalibrierobjekts von dem Deskriptor bezüglich des wenigstens einen Kalibrierobjekts unterscheidet. Auf diese vorteilhafte Weise lassen sich die beiden Kalibrierobjekte und deren Kalibrierelemente voneinander unterscheiden.

In einigen vorteilhaften Ausführungsformen kann das wenigstens eine Kalibrierobjekt oder das weitere Kalibrierobjekt als Stift ausgebildet sein, in welchem die Kalibrierelemente fest eingegossen sind und entlang einer Geraden angeordnet sind.

Alternativ oder zusätzlich kann das wenigstens eine Kalibrierobjekt oder das weitere Kalibrierobjekt in einigen Ausführungsformen als Halterungsvorrichtung für die Kalibrierelemente ausgebildet sein, so dass die Kalibrierelemente in verschiedenen räumlichen Positionen, insbesondere entlang einer Geraden, angeordnet werden können.

Entsprechend können in einigen Ausführungsformen die einzelnen Kalibrierobjekte - also insbesondere das wenigstens eine Kalibrierobjekt und/oder das weitere Kalibrierobjekt - in einer Form des 3-D Kalibrierphantoms fest eingegossen sein oder das 3-D Kalibrierphantom als flexibles Gestell ausgebildet sein, so dass die Kalibrierobjekte auf diesem Gestell flexibel angeordnet werden können. Dabei erhöht ein festes Eingießen vorteilhaft die Robustheit des 3-D Kalibrierphantoms, während ein Ausbilden als Gestell eine flexiblere Anwendung ermöglicht.

Bei einigen Ausführungsformen, bei welchen die Kalibrierobjekte oder zumindest einige davon vier entlang einer Geraden angeordnete Kalibrierelemente aufweisen, weist das 3-D Kalibrierphantom wenigstens drei solcher Kalibrierobjekte - insbesondere Stifte - auf. Auf diese vorteilhafte Weise lässt sich bereits eine Geometriekalibrierung bestimmen, sofern die Kalibrierelemente dieser Kalibrierobjekte nicht koplanar angeordnet sind/werden. Somit wird insbesondere nur eine geringe Gesamtzahl an Kalibrierelementen für die Geometriekalibrierung benötigt.

Insbesondere durch die Verwendung von Stiften als Kalibrierobjekte und/oder durch die Verwendung eines flexiblen Gestells als 3-D Kalibrierphantom lässt sich die Form, Größe und/oder Anzahl an Kalibrierelementen des 3-D Kalibrierphantoms an das jeweils zu untersuchende Objekt, für welches eine geometrische Kalibrierung durchgeführt werden soll, anpassen. Dabei können etwa die Kalibrierobjekte entlang eines langen Zylinders für eine Spiraltrajektorie angeordnet werden, die ein langes Objekt aufnehmen soll. Auch kann die räumliche Ausdehnung des Kalibrierphantoms durch entsprechende Anordnung seiner Kalibrierobjekte an die Größe des zu untersuchenden Objekts angepasst werden, so dass für ein großes Objekt die Kalibrierobjekte weit voneinander beabstandet werden und für ein kleines zu untersuchendes Objekt die Kalibrierobjekte nah beieinander angeordnet werden. Auch lässt sich die Qualität der Bestimmung der Daten für die Geometriekalibrierung dadurch weiter steigern, dass das Kalibrierphantom weitere Kalibrierobjekte oder weitere Kalibrierelemente, welche nicht notwendigerweise einem Kalibrierobjekt und entsprechend einem Deskriptor zugeordnet sein müssen, aufweist. Auch lässt sich durch ein solches 3-D Kalibrierphantom die Güte der Bestimmung der Daten für die Geometriekalibrierung für eine beliebige Richtung und/oder unabhängig von einer Vorzugsrichtung steigern.

Auch lassen sich Kalibrierobjekte mit einer geringen vorbestimmten Anzahl an Kalibrierelementen, etwa weniger als 50, weniger als 30, weniger als 20, weniger als 15 oder nicht mehr als sieben einfacher und/oder mit höherer Genauigkeit fertigen als ein Kalibrierobjekt oder ein Kalibrierphantom mit mehr als diesen Kalibrierelementen.

Die Erfindung betrifft ein Verfahren zum Bestimmen einer Geometriekalibrierung für eine bildgebende Vorrichtung. Bei der bildgebenden Vorrichtung trifft in einer Transmissionsbildgebung eine Strahlung nach Durchgang durch ein 3-D Kalibrierphantom auf einem 2-D Detektor auf zur Erstellung eines 2-D Transmissionsbilds des 3-D Kalibrierphantoms auf. Dabei ist das 3-D Kalibrierphantom gemäß dem zweiten Aspekt der Erfindung ausgebildet. Das Verfahren weist folgendes auf. Das 2-D Transmissionsbild, in dem das wenigstens eine Kalibrierobjekt des 3-D Kalibrierphantoms dargestellt ist, wird bereitgestellt. Mittels eines Verfahrens gemäß dem ersten Aspekt der Erfindung werden im 2-D Transmissionsbild Kalibrierelemente erkannt und zu den physischen Kalibrierelementen des 3-D Kalibrierphantoms zugeordnet. Zudem werden im Verfahren 3-D Anordnungsdaten für die Geometriekalibrierung bereitgestellt, welche die 3-D Anordnung der physischen Kalibrierelemente kennzeichnen. Schließlich werden Projektionsdaten der Geometriekalibrierung, insbesondere eine Projektionsmatrix, für das 2-D Transmissionsbild bestimmt auf Basis der 3-D Anordnungsdaten, auf Basis der den physischen Kalibrierelementen zugeordneten und im 2-D Transmissionsbild erkannten Kalibrierelementen und auf Basis der 2-D Anordnung der erkannten Kalibrierelemente.

Die bereits vorausgehend genannten möglichen Vorteile, Ausführungsformen oder Varianten der vorhergehenden Aspekte Erfindung gelten entsprechend auch für das erfindungsgemäße Verfahren zum Bestimmen einer Geometriekalibrierung.

erfindungsgemäß werden die 3-D Anordnungsdaten bereitgestellt, in dem diese vorbestimmt sind und von einer Datenspeichervorrichtung für die 3-D Anordnungsdaten bezüglich des 3-D Kalibrierphantoms empfangen werden. Auf diese vorteilhafte Weise lässt sich bei vorbestimmten - d. h. insbesondere vorbekannten und/oder vor oder als Teil des Verfahrens physisch gemessenen - 3-D Anordnungsdaten für das verwendete Kalibrierphantom die Bestimmung der Projektionsdaten und damit die Bestimmung der Geometriekalibrierung effizient durchführen und/oder bereits dann durchführen, wenn nur ein 2-D Transmissionsbild vorliegt.

Insbesondere bei solchen mit unbekannten 3-D Anordnungsdaten, weist die Geometriekalibrierung die 3-D Anordnungsdaten und die Projektionsdaten auf. Zur Bestimmung der Geometriekalibrierung wird eines oder werden mehrere zusätzliche 2-D Transmissionsbilder bezüglich zusätzlicher Ansichten des 3-D Kalibrierphantoms bereitgestellt. Zudem werden mittels eines Verfahrens gemäß dem ersten Aspekt der Erfindung jeweilig in den zusätzlichen 2-D Transmissionsbildern erkannte Kalibrierelemente zu den physischen Kalibrierelementen des 3-D Kalibrierphantoms zugeordnet. Schließlich werden die - insbesondere zunächst unbekannten - 3-D Anordnungsdaten bereitgestellt und die jeweiligen Projektionsdaten der Geometriekalibrierung je 2-D Transmissionsbild bestimmt, indem die 3-D Anordnungsdaten so modelliert werden, dass mit diesen 3-D Anordnungsdaten die Abweichungen zwischen den berechneten Deskriptoren für die erkannten Kalibrierelemente der jeweiligen Untermengen der jeweiligen 2-D Transmissionsbilder von den entsprechenden Deskriptoren der physischen Kalibrierelemente minimiert werden. Auf diese vorteilhafte Weise lässt sich die Geometriekalibrierung - lassen sich also insbesondere die 3-D Anordnungsdaten und die Projektionsdaten, insbesondere die Projektionsmatrizen - bestimmen, ohne dass vorab die 3-D Anordnungsdaten bekannt sein müssen. Dadurch lässt sich insbesondere die Güte der Geometriekalibrierung steigern, da diese unabhängig von einer vorausgehenden und gegebenenfalls fehlerbehafteten (insbesondere physischen) Messung der 3-D Anordnung der Kalibrierelemente durchgeführt werden kann. Auch lässt sich auf diese vorteilhafte Weise das Bestimmen der Geometriekalibrierung vereinfachen und/oder flexibler gestalten, da es auf eine exakte, vorab bekannte räumliche Anordnung der Kalibrierelemente nicht ankommt. Vielmehr können Kalibrierelemente des 3-D Kalibrierphantoms vor Durchführung des Verfahrens oder an seinem Anfang - insbesondere abhängig von den zu untersuchenden Objekten - räumlich angeordnet werden, wobei die exakte räumliche Anordnung - und damit die 3-D Anordnungsdaten - im Verfahren bestimmt werden.

In einigen Ausführungsformen weist das Verfahren ein Bestimmen eines zweiten Fehlerwertes auf, welcher die Abweichung jeweils in der 2-D Anordnung eines der erkannten Kalibrierelemente im jeweiligen 2-D Transmissionsbild und der auf Basis der 3-D Anordnungsdaten über die Projektionsdaten berechneten 2-D Anordnung kennzeichnet. Dabei lässt sich in einigen vorteilhaften Varianten die Güte der Geometriekalibrierung mittels der zweiten Fehlerwerte quantifizieren.

In einigen Ausführungsformen mit ersten und zweiten Fehlerwerten wird ein Gesamtfehler auf Basis der ersten Fehlerwerte und der zweiten Fehlerwerte, insbesondere als Summe ihrer Absolutbeträge berechnet.

Bei einigen Ausführungsformen, bei welchen erste oder zweite Fehlerwerte und/oder ein Gesamtfehlerwert berechnet werden, wird das Verfahren iterativ ausgeführt, um wenigstens einen dieser Fehlerwerte zu minimieren. In einigen vorteilhaften Varianten kann das Verfahren als RANSAC-Algorithmus implementiert werden, sodass insbesondere der jeweilige Fehler minimiert wird.

In einigen Ausführungsformen wird zum Bestimmen der Geometriekalibrierung iterativ ein Iterative-Closest-Point-Algorithmus (ICP-Algorithmus) gefolgt von einer nichtlinearen Minimierung des euklidischen Rückprojektionsfehlers - insbesondere ein sogenanntes Bundle Adjustment - ausgeführt. Auf diese Weise lässt sich die Geometriekalibrierung mit besonders hoher Güte - also insbesondere mit besonders hoher Genauigkeit - bestimmen, wobei insbesondere für eine effiziente Implementierung auch auf die Erfahrungen in der Computer Vision mit derartigen Algorithmen zurückgegriffen werden kann.

In einigen Ausführungsformen wird zum Bestimmen der Geometriekalibrierung zunächst iterativ als erster Schritt ein RANSAC-Algorithmus und anschließend als zweiter Schritt eine nichtlinearen Minimierung des euklidischen Rückprojektionsfehlers - insbesondere ein sogenanntes Bundle Adjustment - ausgeführt. Auf diese Weise lässt sich die Geometriekalibrierung zunächst robust anschließend mit besonders hoher Güte bestimmen, wobei insbesondere für eine effiziente Implementierung und/oder eine hohe Robustheit auch auf die Erfahrungen in der Computer Vision mit derartigen Algorithmen zurückgegriffen werden kann.

In einigen Ausführungsformen werden die in den 2-D Transmissionsbildern erkannten Kalibrierelemente zwischen wenigstens einem ersten und einem zweiten 2-D Transmissionsbild mittels einer automatischen Berechnung der Epipolargeometrie einander zugeordnet. Auf diese vorteilhafte Weise lässt sich die Güte der Geometriekalibrierung steigern und/oder die Effizienz bei ihrer Bestimmung steigern, d. h. insbesondere die dafür benötigte Rechenleistung und/oder Rechenzeit reduzieren.

Weitere Vorteile, Merkmale und Anwendungsmöglichkeiten ergeben sich aus der nachfolgenden detaillierten Beschreibung von Ausführungsbeispielen und/oder aus den Figuren.

Kurze Beschreibung der Figuren

Die Erfindung wird nachfolgend unter Bezugnahme auf die Figuren anhand vorteilhafter Ausführungsbeispiele näher erläutert. Gleiche Elemente oder Bauteile der Ausführungsbeispiele werden im Wesentlichen durch gleiche Bezugszeichen gekennzeichnet, falls dies nicht anders beschrieben wird oder sich nicht anders aus dem Kontext ergibt.
Hierzu zeigen, teilweise schematisiert:

Fig. 1: ein 3-D Kalibrierphantom;

Fig. 2: mehrere Kalibrierobjekte für ein 3-D Kalibrierphantom;

Fig. 3: weitere Kalibrierobjekte für ein 3-D Kalibrierphantom;

Fig. 4: ein Flussdiagramm eines Verfahrens zum Bestimmen von Zuordnungsdaten für eine bildgebende Vorrichtung nach einer Ausführungsform;

Fig. 5: ein Flussdiagramm eines Verfahrens zum Bestimmen einer Geometriekalibrierung für eine bildgebende Vorrichtung nach einer Ausführungsform;

Fig. 6: eine Vorrichtung zum Bestimmen einer Geometriekalibrierung für eine bildgebende Vorrichtung; und

Fig. 7: ein kalibrierbares, bildgebendes System.

Die Figuren sind schematische Darstellungen verschiedener Ausführungsformen und/oder Ausführungsbeispiele der vorliegenden Erfindung. In den Figuren dargestellte Elemente und/oder Bauteile sind nicht notwendigerweise maßstabsgetreu dargestellt. Vielmehr sind die verschiedenen in den Figuren dargestellten Elemente und/oder Bauteile derart wiedergegeben, dass ihre Funktion und/oder ihr Zweck dem Fachmann verständlich werden.
In den Figuren dargestellte Verbindungen und Kopplungen zwischen funktionellen Einheiten und Elementen können auch als indirekte Verbindungen oder Kopplungen implementiert werden. Insbesondere können Datenverbindungen drahtgebunden oder drahtlos, also insbesondere als Funkverbindung, ausgebildet sein. Auch können bestimmte Verbindungen, etwa elektrische Verbindungen, etwa zur Energieversorgung, der Übersichtlichkeit halber nicht dargestellt sein.

Detaillierte Beschreibung von Ausführungsbeispielen

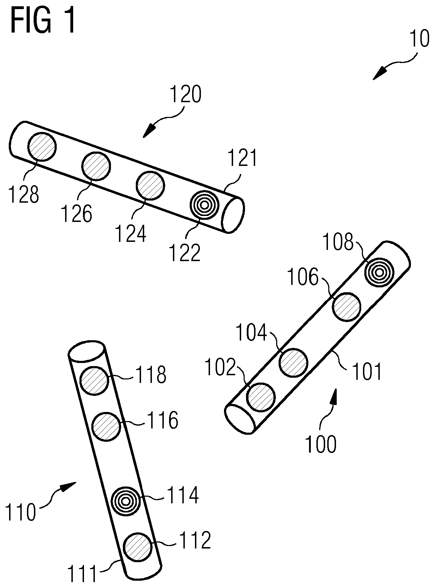

In Fig. 1 ist ein 3-D Kalibrierphantom 10 schematisch dargestellt.
In einem Ausführungsbeispiel weist das 3-D Kalibrierphantom 10 mehrere Kalibrierobjekte 100, 110, 120 - also zumindest wenigstens ein Kalibrierobjekt 100 und ein weiteres Kalibrierobjekt 110 und/oder insgesamt drei Kalibrierobjekte - auf. Jedes der Kalibrierobjekte 100, 110, 120 weist jeweils vier physische Kalibrierelemente 102, 104, 106, 108 bzw. 112, 114, 116, 118 bzw. 122, 124, 126, 128 auf, welche jeweils entlang einer Geraden räumlich angeordnet sind, sowie jeweils eine Halterungsvorrichtung 101, bzw. 111, bzw. 121.
Vorteilhaft können in einigen Varianten die Kalibrierobjekte bezüglich der Geraden, entlang derer ihre jeweiligen Kalibrierelemente räumlich angeordnet sind, windschief zueinander angeordnet sein.
Die physischen Kalibrierelemente 102, 104, 106, 108, 112, 114, 116, 118, 122, 124, 126, 128 weisen ein Material auf, welches eine Strahlung für eine Transmissionsbildgebung streut, oder sind insbesondere aus diesem Material hergestellt. Die Halterungsvorrichtungen 101, 111, 121 sind mit einem oder mit mehreren Materialien so hergestellt, dass diese die Streuung der Strahlung durch die physischen Kalibrierelemente zumindest nicht wesentlich stören, wenn die physischen Kalibrierelemente von der jeweiligen Halterungsvorrichtung gehalten werden. Insbesondere können die Haltungsvorrichtungen dazu aus einem Material hergestellt sein, welches die Strahlung zumindest im Wesentlichen nicht streut.
In einigen Varianten können die Halterungsvorrichtungen 101, 111, 121 aus einem für die Strahlung durchlässigen Kunststoff hergestellt sein und insbesondere daraus bestehen sowie die physischen Kalibrierelemente aus einem Metall oder einer Mischung aus einem Metall und einem Kunststoff hergestellt sein oder daraus bestehen, so dass die Strahlung gestreut wird.
Wie in Fig. 1 dargestellt, unterscheidet sich je eines der physischen Kalibrierelemente 108, 114, 122 von den übrigen physischen Kalibrierelementen der Kalibrierobjekte 100, 110, 120. Dabei unterscheiden sich diese Kalibrierelemente 108, 114, 122 derart, dass diese die Strahlung gegenüber den übrigen physischen Kalibrierelementen anders streuen oder absorbieren, etwa insbesondere weniger streuen. Bei aus einer Mischung von einem Metall und einem Kunststoff hergestellten physischen Kalibrierelementen kann dazu in einigen Varianten für die Kalibrierelemente mit geringerer Streuung 108, 114, 122 beim Mischungsverhältnis ein geringerer Metallanteil und ein höherer Kunststoffanteil verwendet werden.

In einigen Varianten kann zusätzlich oder alternativ auch die Größe der physischen Kalibrierelemente variiert werden und etwa für Kalibrierelemente mit geringerer Streuung oder Absorption eine geringere Größe, d. h., eine geringere räumliche Ausdehnung verwendet werden. Umgekehrt kann etwa für Kalibrierelemente mit größerer Streuung oder Absorption eine größere Größe, d. h., eine größere räumliche Ausdehnung verwendet werden.

In einigen Varianten sind die Halterungsvorrichtungen 101, 111, 121 als Zylinder oder Halbzylinder ausgebildet und weisen jeweils wenigstens vier Aufnahmebereiche je für eines der vier physischen Kalibrierelemente des jeweiligen Kalibrierobjekts auf.

In einigen vorteilhaften Varianten und wie in Fig. 1 dargestellt, kann das gegenüber den übrigen Kalibrierelementen unterschiedliche Kalibrierelement 108, 114, 122 an einer ersten, einer zweiten, einer dritten (nicht dargestellt) oder einer vierten Position bezüglich der Reihenfolge der physischen Kalibrierelemente bei ihrer räumlichen Anordnung entlang der jeweiligen Geraden positioniert sein. So weisen etwa die Kalibrierobjekte 100, 110 jeweils die zumindest im Wesentlichen gleichen Abstände zwischen ihren Kalibrierelementen auf, d. h., der Abstand zwischen dem Kalibrierelement 102 und dem Kalibrierelement 104 entspricht dem Abstand zwischen den Kalibrierelementen 112 und 114, der Abstand zwischen den Kalibrierelementen 102 und 106 entspricht dem Abstand zwischen den Kalibrierelementen 112 und 116 sowie der Abstand zwischen den Kalibrierelementen 102 und 108 entspricht dem Abstand zwischen den Kalibrierelementen 112 und 118, jedoch unterscheiden sich die beiden Kalibrierobjekte 100, 110 dadurch, dass das Kalibrierelement 108 des Kalibrierobjekts 100 an vierter Position und das Kalibrierelement 114 des Kalibrierobjekts 110 an zweiter Position angeordnet ist. Auf diese Weise lassen sich ansonsten zumindest im Wesentlichen gleiche Kalibrierobjekte voneinander unterscheiden und/oder es lässt sich eine Reihenfolge der Kalibrierelemente des jeweiligen Kalibrierobjekts festlegen.

Eine weitere Unterscheidungsmöglichkeit für Kalibrierobjekte ist eine unterschiedliche räumliche Anordnung, d. h. insbesondere unterschiedliche räumliche Abstände ihrer jeweiligen Kalibrierelemente. So sind etwa die Kalibrierelemente des Kalibrierobjekts 120 gleich beabstandet, während die Abstände zwischen den Kalibrierelementen des Kalibrierobjekts 100 unterschiedlich sind.

Als Deskriptor für das jeweilige Kalibrierobjekt 100, 110, 120 lässt sich, insbesondere durch die Anordnung der jeweils vier Kalibrierelemente entlang einer Geraden, das Doppelverhältnis basierend auf den Abständen der jeweiligen Kalibrierelemente untereinander bestimmen. Dabei kann der jeweilige Deskriptor insbesondere weitere Merkmale, insbesondere die Reihenfolge der Kalibrierelemente und/oder die Anordnung der Kalibrierelemente entlang einer Geraden aufweisen.

Dieses Doppelverhältnis ist bei einer Transmissionsbildgebung mittels einer bildgebenden Vorrichtung, bei welcher eine Strahlung nach Durchgang durch das 3-D Kalibrierphantom 10 auf einen 2-D Detektor auftrifft zur Erstellung eines 2-D Transmissionsbildes des 3-D Kalibrierphantoms 10, projektiv invariant. Eine solche Transmissionsbildgebung kann bezüglich der Abbildung des jeweiligen dreidimensionalen Objekts, d. h. hier insbesondere des 3-D Kalibrierphantoms, auf das 2-D Transmissionsbild verschiedene Transformationen aufweisen, insbesondere eine rigide Transformation, eine Ähnlichkeitstransformation, eine affine Transformation, eine pseudoaffine Transformation oder im allgemeinen eine projektive Transformation. Ein Vorteil der Verwendung des Doppelverhältnisses als Deskriptor oder als Teil des Deskriptors kann also insbesondere darin liegen, dass dieses Doppelverhältnis bezüglich der Transformationen projektiv invariant ist, so dass dieses unabhängig von der jeweiligen konkreten Transformation bei der Transmissionsbildgebung bestimmt werden kann und dabei einen gleich bleibenden - also invarianten - Wert liefert, wodurch das jeweilige Kalibrierobjekt über seinen Deskriptor, d. h. insbesondere über den jeweiligen Wert des Doppelverhältnisses für dieses Kalibrierobjekt identifizierbar ist. Demnach lassen sich auch die Kalibrierelemente eines so identifizierten Kalibrierobjekts ihren jeweiligen Abbildungen im 2-D Transmissionsbild zuordnen und/oder umgekehrt, wobei es in einigen Varianten erforderlich sein kann, die Reihenfolge der Kalibrierelemente des jeweiligen Kalibrierobjekts festzulegen und vorteilhaft etwa mittels eines Unterscheidungskriteriums für diese Kalibrierelemente zu bestimmen.

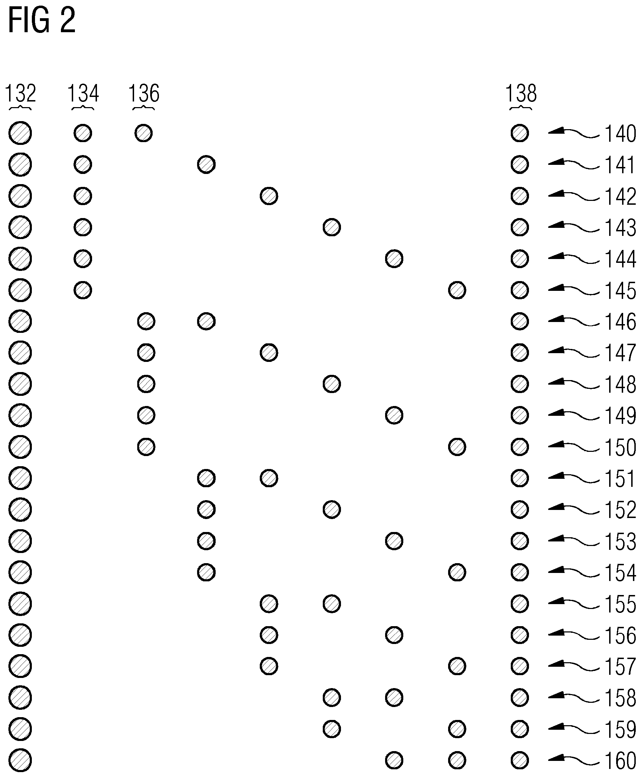

Fig. 2 zeigt schematisch mehrere Kalibrierobjekte 140, 141, 142,... 160 für ein 3-D Kalibrierphantom.

In einem Ausführungsbeispiel weisen die Kalibrierobjekte 140-160 jeweils vier Kalibrierelemente 132, 134, 136, 138 auf. Die Kalibrierelemente 132, 134, 136, 138 bestehen zumindest im Wesentlichen aus einem Material, das die Strahlung absorbiert. Zudem weist jeweils die räumliche Ausdehnung des jeweiligen Kalibrierelements 132 (für das jeweilige Kalibrierobjekt 140-160) eine größere räumliche Ausdehnung auf als die übrigen Kalibrierelemente 134, 136, 138. Betrachtet man in Fig. 2 die Kalibrierelemente für das jeweilige Kalibrierobjekt von links nach rechts so ist das Kalibrierelement 132, welches sich durch seine räumliche Ausdehnung unterscheidet, an einer ersten Position angeordnet.

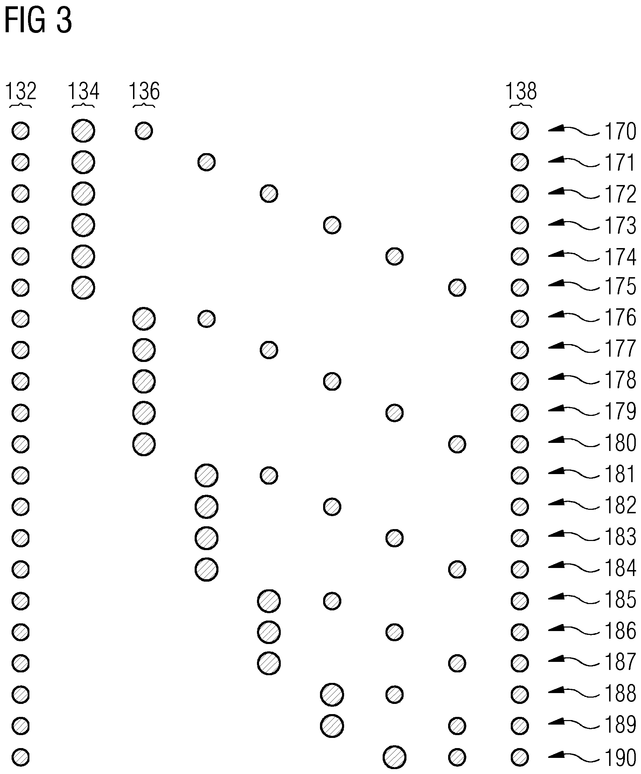

Entsprechend zeigt Fig. 3 weitere Kalibrierobjekte 170, 171,... 190 für ein 3-D Kalibrierphantom.

Auch diese Kalibrierobjekte 170-190 weisen in einem Ausführungsbeispiel jeweils vier Kalibrierelemente 132, 134, 136, 138 auf. Die Kalibrierobjekte aus Fig. 3 unterscheiden sich von den Kalibrierobjekten aus Fig. 2 darin, dass das jeweilige Kalibrierelement 134 bei Fig. 3 gegenüber den anderen Kalibrierelementen 132, 136, 138 unterschiedlich ist, insbesondere eine größere räumliche Ausdehnung aufweist, und dieses jeweilige Kalibrierelement 134 an zweiter Position angeordnet ist.

Bei einem Deskriptor, insbesondere einem Doppelverhältnis, lässt sich dieser Unterschied zwischen den Kalibrierobjekten aus Fig. 2 und Fig. 3 dadurch kennzeichnen, dass das Doppelverhältnis für Kalibrierobjekte mit dem unterschiedlichen Kalibrierelement an erster Position positiv und das Doppelverhältnis für Kalibrierobjekte mit dem unterschiedlichen Kalibrierelement an zweiter Position mit einem negativen Wert codiert wird.

Für die in Fig. 2 dargestellten Kalibrierobjekte ergibt sich als codierter Wert für das Doppelverhältnis für den Deskriptor folgendes:

| Kalibrierobjekt | Doppelverhältnis |
|---|---|
| 140 | 2 |
| 141 | 4 |
| 142 | 7 |
| 143 | 11 |
| 144 | 21 |
| 145 | 49 |
| 146 | 1 |
| 147 | 3 |
| 148 | 5 |
| 149 | 9 |
| 150 | 21 |
| 151 | 1 |
| 152 | 2 |
| 153 | 5 |
| 154 | 11 |
| 155 | 1 |
| 156 | 3 |
| 157 | 7 |
| 158 | 1 |
| 159 | 4 |
| 160 | 2 |

Für die in Fig. 3 dargestellten Kalibrierobjekte ergibt sich als codierter Wert für das Doppelverhältnis für den Deskriptor folgendes:

| Kalibrierobjekt | Doppelverhältnis |
|---|---|
| 170 | -2 |
| 171 | -4 |
| 172 | -7 |
| 173 | -11 |
| 174 | -21 |
| 175 | -49 |
| 176 | -1 |
| 177 | -3 |
| 178 | -5 |
| 179 | -9 |
| 180 | -21 |
| 181 | -1 |
| 182 | -2 |
| 183 | -5 |

(fortgesetzt)

| Kalibrierobjekt | Doppelverhältnis |
|---|---|
| 184 | -11 |
| 185 | -1 |
| 186 | -3 |
| 187 | -7 |
| 188 | -1 |
| 189 | -4 |
| 190 | -2 |

Vorteilhaft weist dabei in einigen Varianten ein 3-D Kalibrierphantom solche Kalibrierobjekte auf, die sich im Wert ihres Doppelverhältnisses zumindest bezüglich ihres Vorzeichens unterscheiden, so dass die einzelnen Kalibrierobjekte insbesondere anhand ihres Deskriptors eindeutig zugeordnet werden können, insbesondere also ein starker Deskriptor vorliegt.

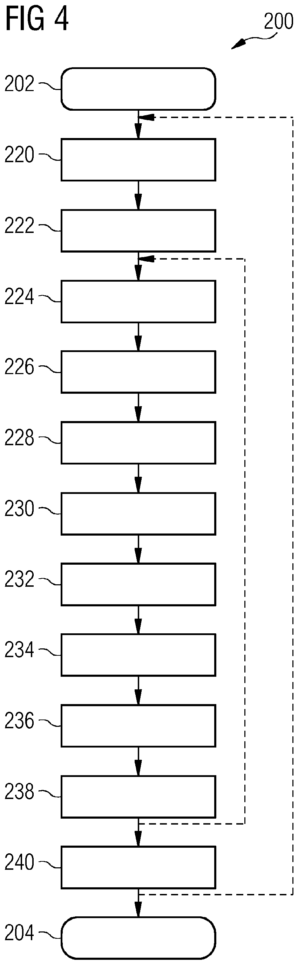

In Fig. 4 ist ein Flussdiagramm eines Verfahrens 200 zum Bestimmen von Zuordnungsdaten für eine bildgebende Vorrichtung nach einer Ausführungsform der vorliegenden Erfindung dargestellt. Bei einer solchen bildgebenden Vorrichtung trifft zur Erstellung eines 2-D Transmissionsbilds eines 3-D Kalibrierphantoms in einer Transmissionsbildgebung eine Strahlung nach Durchgang durch das 3-D Kalibrierphantom auf einem 2-D Detektor auf. Dabei kann das 3-D Kalibrierphantom insbesondere einem der Kalibrierphantome gemäß der Figuren 1, 2 oder 3 entsprechen.

In einem Ausführungsbeispiel weist das Verfahren 200 die Verfahrensschritte 220, 222, 224, 226, 228, 230, 232, 234, 236, 238 und 240 auf. Das Verfahren 200 beginnt bei dem Verfahrensstart 202 und endet bei dem Verfahrensende 204, wobei einer oder mehrere Verfahrensschritte, insbesondere eine Sequenz von Verfahrensschritten, und vorzugsweise das gesamte Verfahren wiederholt ausgeführt werden können.

Im Verfahrensschritt 220 wird das 2-D Transmissionsbild, in dem das wenigstens eine Kalibrierobjekt des 3-D Phantoms dargestellt ist, bereitgestellt.

Im Verfahrensschritt 222 werden zur Bestimmung von im 2-D Transmissionsbild erkannten Kalibrierelementen und ihrer 2-D Anordnung die in dem 2-D Transmissionsbild dargestellten physischen Kalibrierelemente erkannt.

In einigen Varianten können die Kalibrierelemente als Kugeln oder Perlen ausgebildet sein. Auch können in einigen Varianten die im 2-D Transmissionsbild dargestellten physischen Kalibrierelemente, insbesondere wenn sie kugelförmig oder perlenförmig sind, mittels eines sogenannten "Blob Detectors" erkannt werden. Dabei kann in einigen Varianten auch die räumliche bzw. im 2-D Transmissionsbild die zweidimensionale Ausdehnung des jeweils erkannten Kalibrierelements als Unterscheidungskriterium, insbesondere mittels eines solchen "Blob Detectors", bestimmt werden.

In einigen Varianten, bei welchen das wenigstens eine Kalibrierobjekt des 3-D Kalibrierphantoms ein Kalibrierelement mit einer größeren räumlichen Ausdehnung aufweist, kann die räumliche Ausdehnung bzw. die zweidimensionale Ausdehnung im 2-D Transmissionsbild - zumindest relativ zu anderen Kalibrierelementen zumindest derselben Untermenge - über eine sogenannte "Fast Radial Symmetry Transform", welche zweifach angewendet wird, und einem Entfernen von Kalibrierelementen, welche eine zu geringe Ausdehnung aufweisen und/oder zu nah an anderen Kalibrierelementen liegen, die 2-D Anordnung der Kalibrierelemente mit größerer Ausdehnung und der Kalibrierelemente mit kleinerer Ausdehnung bestimmt werden.

Im Verfahrensschritt 224 werden Untermengen der erkannten Kalibrierelemente derart bestimmt, dass die Anzahl an Kalibrierelementen bei den Untermengen jeweils der vorbestimmten Anzahl an physischen Kalibrierelementen des wenigstens einen Kalibrierobjekts entspricht. Insbesondere bei Kalibrierobjekten, deren Kalibrierelemente entlang einer Geraden angeordnet sind, können Untermengen dabei des Weiteren so bestimmt werden, dass die erkannten Kalibrierelemente jeweils einer der Untermengen auch im 2-D Transmissionsbild entlang einer Geraden angeordnet sind.

Im Verfahrensschritt 226 wird jeweils der Deskriptor bezüglich der Kalibrierelemente je Untermenge berechnet. In einigen Varianten, bei welchen die Kalibrierelemente des wenigstens einen Kalibrierobjekts entlang einer geraden angeordnet sind und das Kalibrierobjekt wenigstens vier und insbesondere genau vier Kalibrierelemente aufweist, kann der Deskriptor oder ein Teil davon über das Doppelverhältnis, wie oben beschrieben, bestimmt werden.

Da das Doppelverhältnis insbesondere von der Reihenfolge der Kalibrierelemente innerhalb jeweils der jeweiligen Untermenge abhängen kann - d. h. insbesondere sich für verschiedene Permutationen innerhalb einer Untermenge unterschiedliche Werte für das Doppelverhältnis ergeben - kann es vorteilhaft sein, die Reihenfolge der Kalibrierelemente mittels eines Unterscheidungskriteriums für die einzelnen Kalibrierelemente des wenigstens einen Kalibrierobjekts festzulegen.

In einigen Varianten wird, insbesondere um die Reihenfolge der Kalibrierelemente festzulegen, im Verfahrensschritt 228 die Ausdehnung der Kalibrierelemente als das Unterscheidungskriterium auf dem 2-D Transmissionsbild je erkannten Kalibrierelement bestimmt.

Im Verfahrensschritt 230 werden, basierend darauf, jene Kalibrierelemente bestimmt, welche sich gegenüber den übrigen Kalibrierelementen dieser Untermenge durch eine größere Ausdehnung unterscheiden, bestimmt.

Im Verfahrensschritt 232 wird die Reihenfolge der Kalibrierelemente für jede Untermenge mittels des Unterscheidungskriteriums, also insbesondere mittels der Ausdehnung im 2-D Transmissionsbild, festgelegt, so dass etwa die jeweilige Untermenge so geordnet wird, dass bei äußerer Lage des erkannten Kalibrierelements, welches sich aufgrund seiner Ausdehnung von den übrigen dieser Untermenge unterscheidet, dieses Kalibrierelement an erster Position ist und dieses unterschiedliche Kalibrierelement, wenn es in einer inneren Lage ist - d. h., andere Kalibrierelemente entlang der Geraden noch weiter außen liegen -, an zweiter Position ist. Dabei wird in einigen Varianten von einer pseudo-affinen Abbildung des 3-D Kalibrierphantoms auf das 2-D Transmissionsbild ausgegangen, sodass insbesondere die Anordnung oder Reihenfolge entlang einer Gerade unter der Abbildung erhalten bleibt.

Alternativ oder zusätzlich zum Festlegen der Reihenfolge können auch weitere vorbekannte Daten zum eindeutigen Bestimmen des Deskriptors verwendet werden oder das Verfahren iterativ angewandt werden, so dass insbesondere etwaige Deskriptoren für alle möglichen Permutationen oder zumindest einen wesentlichen Teil der Permutationen bestimmt werden.

In einigen Varianten werden im Verfahrensschritt 234 die Deskriptoren der (physischen) Kalibrierobjekte bereitgestellt, indem diese von einer Datenspeichervorrichtung empfangen werden. Alternativ oder zusätzlich können in einigen Varianten diese Deskriptoren durch ein iteratives Ausführen des Verfahrens im Rahmen einer Optimierung - etwas mittels RANSAC-Algorithmus - bestimmt werden.

Im Verfahrensschritt 236 wird jeweils je Untermenge ein erster Fehlerwert berechnet, welcher die Abweichung zwischen dem für diese Untermenge berechneten Deskriptor und dem Deskriptor des wenigstens einen Kalibrierobjekts kennzeichnet. Bei einigen Varianten, bei welchen das Verfahren iterativ ausgeführt wird, wird dabei ein Gesamtfehler dieser ersten Fehler gebildet und dieser für das wenigstens eine Kalibrierobjekt und weitere Kalibrierobjekte und/oder für das 2-D Transmissionsbild und weitere Transmissionsbilder optimiert.

Im Verfahrensschritt 238 werden die Zuordnungsdaten bestimmt, indem die erkannten Kalibrierelemente einer der Untermengen den physischen Kalibrierelementen des wenigstens einen Kalibrierobjekts abhängig davon zugeordnet werden, ob der jeweilige erste Fehlerwert geringer als ein erster Grenzwert ist. In einigen Varianten kann dabei dieser erste Grenzwert vorbestimmt sein. Alternativ oder zusätzlich kann in einigen Varianten dieser erste Grenzwert dynamisch sein und von den ersten Fehlerwerten bezüglich mehrerer der Untermengen abhängig sein, so dass insbesondere bei mehreren Kalibrierobjekten die Zuordnung der Kalibrierelemente optimiert wird und so insbesondere der Gesamtfehler, welcher auf den ersten Fehlerwerten basiert, minimiert wird.

Eine solche iterative Optimierung kann in einigen Varianten vorteilhaft mittels den sogenannten RANSAC-Algorithmus ausgeführt werden, wobei insbesondere die Verfahrensschritte 224-236 und insbesondere bei Verwendung mehrerer 2-D Transmissionsbilder auch der Verfahrensschritt 238 wiederholt und für verschieden bestimmte Untermengen iterativ ausgeführt werden, insbesondere bis ein Gesamtfehler unter einem vorbestimmten Grenzwert für den Gesamtfehler liegt oder eine bestimmte Anzahl an Iterationen ausgeführt worden ist oder statistisch kein wesentlich geringerer Gesamtfehler bei weiteren Iterationen zu erwarten ist.

Schließlich werden im Verfahrensschritt 240 die Zuordnungsdaten bereitgestellt. In einigen Varianten können die Zuordnungsdaten dabei mittels einer Datenschnittstelle ausgegeben werden.

Ein Vorteil dieses Verfahrens und der so bestimmten Zuordnungsdaten kann insbesondere daran liegen, dass den im 2-D Bild erkannten Kalibrierelementen die physischen Kalibrierelemente des wenigstens einen Kalibrierobjekts zugeordnet werden können, wodurch insbesondere das sogenannte Korrespondenzproblem gelöst werden kann und womit sich Algorithmen aus der Computer Vision zur Bestimmung von Abbildungseigenschaften und/oder Projektionseigenschaften auf die Transmissionsbildgebung übertragen lassen und so insbesondere zur Bestimmung der Geometriekalibrierung verwendet werden können.

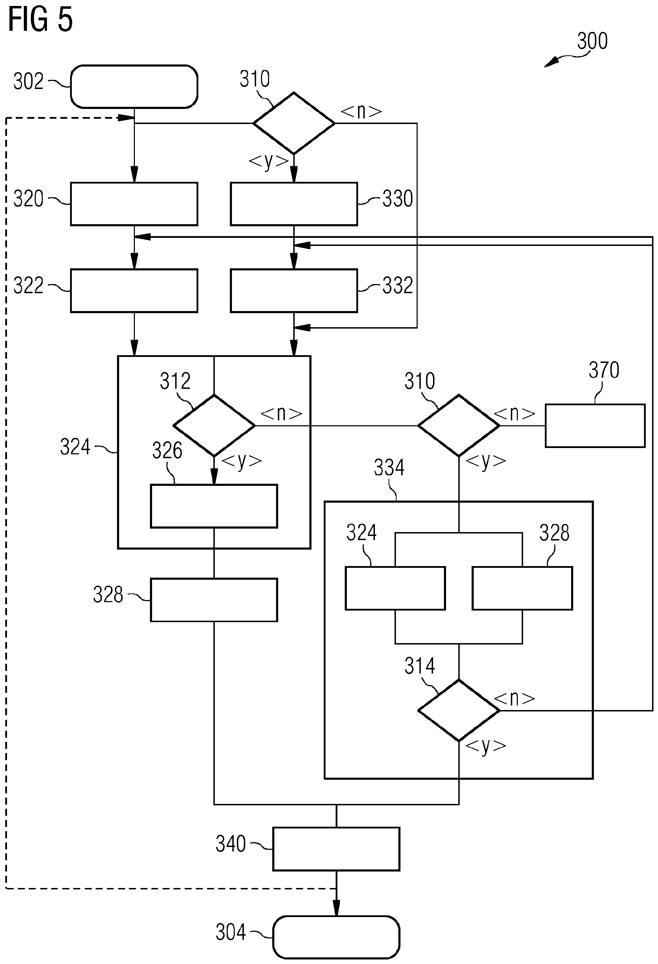

In Fig. 5 ist ein Flussdiagramm eines Verfahrens 300 zum Bestimmen einer Geometriekalibrierung für eine bildgebende Vorrichtung nach einer Ausführungsform der vorliegenden Erfindung dargestellt. Bei der bildgebenden Vorrichtung trifft in einer Transmissionsbildgebung eine Strahlung nach Durchgang durch ein 3-D Kalibrierphantom, welches gemäß einer Ausführungsform des 3-D Kalibrierphantoms der vorliegenden Erfindung und insbesondere gemäß einer Ausführungsform bezüglich der vorhergehenden Figuren ausgebildet ist, auf einen 2-D Detektor auf zur Erstellung eines 2-D Transmissionsbilds des 3-D Kalibrierphantoms.

In einem Ausführungsbeispiel weist das Verfahren 300 die Verfahrensschritte 320, 322, 324, 326, 328, 330, 332, 334, 340 und 370 sowie die Verfahrensbedingungen 310, 312 und 314 auf. Das Verfahren 300 beginnt bei dem Verfahrensstart 302 und endet bei dem Verfahrensende 304, wobei einer oder mehrere Verfahrensschritte, insbesondere eine Sequenz von Verfahrensschritten, und vorzugsweise das gesamte Verfahren wiederholt ausgeführt werden können.

Im Verfahrensschritt 320 wird das 2-D Transmissionsbild, in dem das wenigstens eine Kalibrierobjekt des 3-D Kalibrierphantoms dargestellt ist, bereitgestellt.

Im Verfahrensschritt 322 wird ein Verfahren zum Bestimmen von Zuordnungsdaten nach einer Ausführungsform der vorliegenden Erfindung und insbesondere nach einer Ausführungsform bezüglich der Fig. 4 ausgeführt, um im 2-D

EP 3 569 147 B1

Transmissionsbild Kalibrierelemente zu erkennen und den physischen Kalibrierelementen des 3-D Kalibrierphantoms zuzuordnen.

Bei der Verfahrensbedingung 310 wird geprüft, ob das Verfahren für das eine 2-D Transmissionsbild ausgeführt werden soll, oder, ob das Verfahren auch für zusätzliche 2-D Transmissionsbilder ausgeführt werden soll.

Falls dies der Fall ist - was im Flussdiagramm durch <y> symbolisiert ist -, werden im Verfahrensschritt 330 eines oder mehrere zusätzliche 2-D Transmissionsbilder bezüglich zusätzlicher Ansichten des 3-D Kalibrierphantoms bereitgestellt. Zudem werden im Verfahrensschritt 332 den physischen Kalibrierelementen des 3-D Kalibrierphantoms den jeweils in den jeweiligen zusätzlichen 2-D Transmissionsbildern erkannten Kalibrierelementen entsprechend dem Verfahrensstart 322 zugeordnet.

Falls dies nicht der Fall ist - also das Verfahren nur für das eine 2-D Transmissionsbild ausgeführt werden soll, was in Fig. 5 durch <n> symbolisiert ist - werden die Verfahrensschritte 330 und 332 nicht ausgeführt.

Im Verfahrensschritt 324 werden 3-D Anordnungsdaten für die Geometriekalibrierung, welche die 3-D Anordnung der physischen Kalibrierelemente kennzeichnen, bereitgestellt. Dabei wird gemäß Verfahrensbedingungen 312 unterschieden, ob die 3-D Anordnungsdaten vorbekannt und/oder gespeichert sind.

Falls dies der Fall ist, werden die 3-D Anordnungsdaten im Verfahrensschritt 326 von einer Datenspeichervorrichtung für die 3-D Anordnungsdaten bezüglich des 3-D Kalibrierphantoms empfangen. Falls dies nicht der Fall ist - symbolisiert durch <n> -, wird mit der Verfahrensbedingungen 310 geprüft, ob zusätzliche 2-D Transmissionsbilder vorliegen. Falls dies nicht der Fall ist, wird im Verfahrensschritt 370 ein Fehlersignal ausgegeben, welches unzureichende Daten kennzeichnet, woraufhin das Verfahren insbesondere angehalten oder beendet werden kann oder erneut - etwa mit weiteren Daten/2-D Transmissionsbildern - ausgeführt werden kann.

Falls zusätzliche 2-D Transmissionsbilder vorliegen, wird der Verfahrensschritt 334 ausgeführt.

Im Verfahrensschritt 334 werden parallel die 3-D Anordnungsdaten gemäß einem alternativen Verfahrensschritt 324 bereitgestellt und die Projektionsdaten gemäß einem alternativen Verfahrensschritt 328 bestimmt, indem die 3-D Anordnungsdaten so modelliert werden, dass mit diesen 3-D Anordnungsdaten die Abweichungen zwischen den berechneten Deskriptoren für die erkannten Kalibrierelemente der jeweiligen Untermengen der jeweiligen 2-D Transmissionsbilder von den entsprechenden Deskriptoren der physischen Kalibrierelemente minimiert werden, wobei bei der Verfahrensbedingung 314 geprüft wird, ob eine hinreichende Minimierung vorliegt, und, falls dies nicht der Fall ist, die Verfahrensschritte ab Verfahrensschritt 322 sowie die Verfahrensschritte ab Verfahrensschritt 332 erneut ausgeführt werden.

In einigen Varianten kann eine derartige Minimierung vorteilhaft mittels des RANSAC-Algorithmus ausgeführt werden.

Ein Vorteil eines solchen iterativen Verfahrens kann insbesondere darin liegen, dass die 3-D Anordnung der Kalibrierelemente des 3-D Kalibrierphantoms nicht vorbekannt sein muss, sondern durch das iterative Verfahren selbst bestimmt werden kann. Auf diese vorteilhafte Weise lässt sich eine erhöhte Genauigkeit erzielen und/oder es lässt sich eine Abhängigkeit von den vorbekannten Werten und Daten für die 3-D Anordnung der Kalibrierelemente, welche selbst mit einer Unsicherheit behaftet sein können, vermeiden. Zur weiteren Bestimmung der Geometriekalibrierung, also insbesondere der 3-D Anordnungsdaten, der Zuordnung zwischen den Kalibrierelementen des 3-D Kalibrierphantoms und der erkannten Kalibrierelemente in den 2-D Transmissionsbildern sowie entsprechender Projektionsmatrizen, können weitere Daten, insbesondere ein Skalierungsfaktor, bereitgestellt werden.

Sofern die 3-D Anordnungsdaten vorbekannt sind, also Verfahrensbedingung 312 erfüllt ist, werden nach dem Verfahrensschritt 326 im Verfahrensschritt 328 die Projektionsdaten der Geometriekalibrierung, also insbesondere eine Projektionsmatrix, für das 2-D Transmissionsbild auf Basis der 3-D Anordnungsdaten, der den physischen Kalibrierelementen zugeordneten und im 2-D Transmissionsbild erkannten Kalibrierelemente und der 2-D Anordnung der erkannten Kalibrierelemente bestimmt. Hierbei kann in einigen Varianten das Verfahren ebenfalls zur Optimierung wiederholt ausgeführt werden. Dabei müssen jedoch die 3-D Anordnungsdaten nicht bestimmt werden, so dass sich eine schnellere Konvergenz erzielen lässt.

Schließlich werden im Verfahrensschritt 340 Daten bezüglich der mittels dieses Verfahrens bestimmten Geometriekalibrierung, also insbesondere je eine Projektionsmatrix je 2-D Transmissionsbild, mittels einer Datenschnittstelle ausgegeben. Anhand dieser Geometriekalibrierung lassen sich aus 2-D Transmissionsbildern eines zu untersuchenden Objekts dreidimensionale Daten bezüglich dieses zu untersuchenden Objekts rekonstruieren, wobei insbesondere Abbildungsfehler der jeweiligen bildgebenden Vorrichtung herausgerechnet werden können.

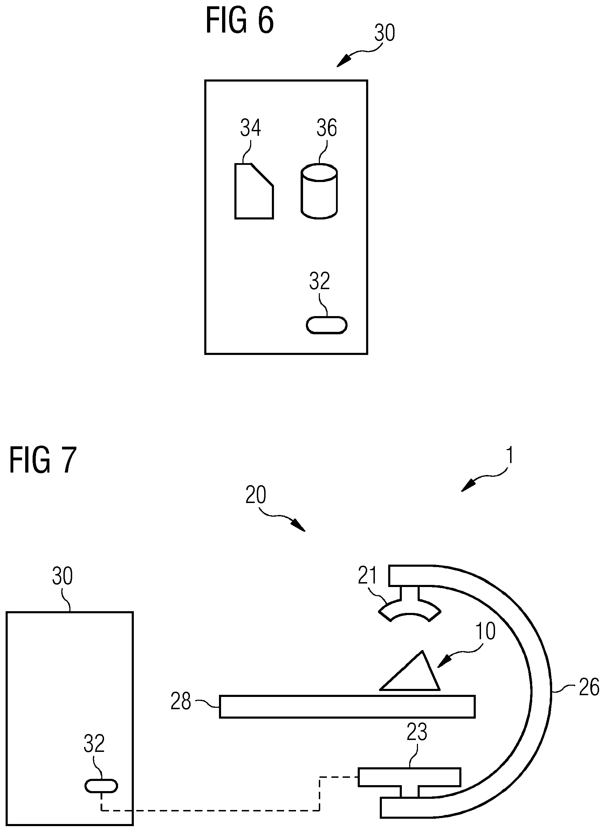

Fig. 6 zeigt schematisch eine Vorrichtung 30 zum Bestimmen einer Geometriekalibrierung für eine bildgebende Vorrichtung, bei welcher in einer Transmissionsbildgebung eine Strahlung nach Durchgang durch ein 3-D Kalibrierphantom auf einen 2-D Detektor zur Erstellung eines 2-D Transmissionsbilds des 3-D Kalibrierphantoms auftrifft. Dabei ist das 3-D Kalibrierphantom gemäß einer der Figuren 1, 2 und 3 ausgebildet.

In einem Beispiel weist die Vorrichtung 30 eine Datenverarbeitungsvorrichtung 34, eine Datenspeichervorrichtung 36 und eine Datenschnittstelle 32 auf. Dabei ist die Datenschnittstelle 32 zum Empfangen von 2-D Transmissionsbildern von der bildgebenden Vorrichtung eingerichtet. Zudem ist die Vorrichtung 30 eingerichtet, ein Verfahren zum

14

Bestimmen einer Geometriekalibrierung für eine bildgebende Vorrichtung gemäß einer Ausführungsform der vorliegenden Erfindung und insbesondere gemäß einer Ausführungsform bezüglich Fig. 5 auszuführen.

In Fig. 7 ist ein kalibrierbares, bildgebendes System schematisch dargestellt.

Das System 1 weist eine bildgebende Vorrichtung 20 und eine Vorrichtung 30 zum Bestimmen der Geometriekalibrierung auf. Die Vorrichtung 30 zum Bestimmen der Geometriekalibrierung ist insbesondere gemäß einer Ausführungsform bezüglich Fig. 6 ausgebildet. In einigen Varianten kann die bildgebende Vorrichtung 20 als Computertomographievorrichtung ausgebildet sein.

In einigen Varianten kann das System 1 zudem ein 3-D Kalibrierphantom 10 gemäß einer Ausführungsform der vorliegenden Erfindung und insbesondere gemäß einer Ausführungsform bezüglich der Figuren 1, 2, 3 aufweisen. In anderen Varianten kann das System 1 das 3-D Kalibrierphantom 10 nicht aufweisen, aber eingerichtet sein, nach Bereitstellung des 3-D Kalibrierphantoms 10 eines oder mehrere 2-D Transmissionsbilder des 3-D Kalibrierphantoms aufzunehmen.

In einem Ausführungsbeispiel weist die bildgebende Vorrichtung 20 eine Strahlungsquelle 21 zum Erzeugen einer Strahlung und einen 2-D Detektor 23 auf. Dabei können die Strahlungsquelle 21 und der 2-D Detektor 23 entlang einer Halterungsvorrichtung 26 - insbesondere ausgebildet als ein sogenannter C-Bogen - angeordnet werden. Zudem kann ein 3-D Kalibrierphantom 10 auf eine Trägervorrichtung 28 - etwa einen Tisch - angeordnet werden. Beim Durchführen einer Transmissionsbildgebung mittels der bildgebenden Vorrichtung 20 wird ein 2-D Transmissionsbild des 3-D Kalibrierphantoms 10 erstellt, indem die Strahlung aus der Strahlungsquelle 21 nach Durchgang durch das 3-D Kalibrierphantom 10 auf dem 2-D Detektor 23 auftrifft.

Zum Bereitstellen des aufgenommenen 2-D Transmissionsbilds weist die bildgebende Vorrichtung 20, insbesondere der 2-D Detektor 23, eine Datenschnittstelle auf, die ausgebildet ist eine Datenverbindung mit der Datenschnittstelle 32 der Vorrichtung 30 zum Bestimmen der Geometriekalibrierung auszubilden.

Während Ausführungsbeispiele insbesondere unter Bezugnahme auf die Figuren detailliert beschrieben wurden, sei darauf hingewiesen, dass eine Vielzahl von Abwandlungen möglich ist.

## Patentansprüche

1. Verfahren (300) zum Bestimmen einer Geometriekalibrierung für eine bildgebende Vorrichtung (20), bei welcher in einer Transmissionsbildgebung eine Strahlung nach Durchgang durch ein 3-D Kalibrierphantom (10) auf einem 2-D Detektor (23) auftrifft zur Erstellung eines 2-D Transmissionsbilds des 3-D Kalibrierphantoms (10), wobei das 3-D Kalibrierphantom (10) wenigstens ein Kalibrierobjekt (100) mit einer vorbestimmten Anzahl an physischen Kalibrierelementen (102, 104, 106, 108) aufweist, welche derart räumlich angeordnet sind, dass ein auf der räumlichen Anordnung basierender Deskriptor unter der Transmissionsbildgebung projektiv invariant ist, wobei die physischen Kalibrierelemente (102, 104, 106, 108; 112, 114, 116, 118) ein Material aufweisen, welches für die Strahlung opak ist und/oder welches die Strahlung absorbiert oder streut, wobei das Verfahren aufweist:

   - Bereitstellen (320) des 2-D Transmissionsbilds, in dem das wenigstens eine Kalibrierobjekt des 3-D Kalibrierphantoms dargestellt ist;
   - Zuordnen (322) von im 2-D Transmissionsbild erkannten Kalibrierelementen zu den physischen Kalibrierelementen des 3-D Kalibrierphantoms;
   - Bereitstellen (324) von 3-D Anordnungsdaten für die Geometriekalibrierung, welche die 3-D Anordnung der physischen Kalibrierelemente kennzeichnen; und
   - Bestimmen (328) von Projektionsdaten der Geometriekalibrierung, insbesondere einer Projektionsmatrix, für das 2-D Transmissionsbild auf Basis der 3-D Anordnungsdaten, der den physischen Kalibrierelementen zugeordneten und im 2-D Transmissionsbild erkannten Kalibrierelemente und der 2-D Anordnung der erkannten Kalibrierelemente;

   wobei das Zuordnen (322) aufweist:

   - Erkennen (222) der in dem 2-D Transmissionsbild dargestellten physischen Kalibrierelemente (102, 104, 106, 108) zur Bestimmung von im 2-D Transmissionsbild erkannten Kalibrierelementen und ihrer 2-D Anordnung;
   - Bestimmen (224) von Untermengen der erkannten Kalibrierelemente, deren Anzahl an Kalibrierelementen jeweils der vorbestimmten Anzahl an physischen Kalibrierelementen (102, 104, 106, 108) des wenigstens einen Kalibrierobjekt (100) entspricht;
   - Berechnen (226) jeweils des Deskriptors bezüglich der Kalibrierelemente je Untermenge; und
   - Bestimmen (238) der Zuordnungsdaten, indem die erkannten Kalibrierelemente jener Untermenge den physischen Kalibrierelementen (102, 104, 106, 108) des wenigstens einen Kalibrierobjekts (100) zugeordnet werden, für welche der berechnete Deskriptor und der Deskriptor bezüglich der physischen Kalibrierelemente am wenigsten voneinander abweichen;

und wobei das Verfahren (300) des Weiteren aufweist:

- Bereitstellen (330) eines oder mehrerer zusätzlicher 2-D Transmissionsbilder bezüglich zusätzlicher Ansichten des 3-D Kalibrierphantoms;
- jeweiliges Zuordnen (332) von im jeweiligen zusätzlichen 2-D Transmissionsbild erkannten Kalibrierelementen zu den physischen Kalibrierelementen des 3-D Kalibrierphantoms; und
- Bereitstellen (324) der 3-D Anordnungsdaten und Bestimmen (328) der jeweiligen Projektionsdaten der Geometriekalibrierung je 2-D Transmissionsbild, indem die 3-D Anordnungsdaten so modelliert (334) werden, dass mit diesen 3-D Anordnungsdaten die Abweichungen zwischen den berechneten Deskriptoren für die erkannten Kalibrierelemente der jeweiligen Untermengen der jeweiligen 2-D Transmissionsbilder von den entsprechenden Deskriptoren der physischen Kalibrierelemente minimiert werden.

2. Verfahren (300) gemäß Anspruch 1, für welches das 3-D Kalibrierphantom (10) eine Vielzahl an Kalibrierobjekten aufweist, wobei das wenigstens eine Kalibrierobjekt (100) und ein weiteres Kalibrierobjekt (110, 120) jeweils Teil der Vielzahl an Kalibrierobjekten sind, und wobei die Kalibrierobjekte der Vielzahl an Kalibrierobjekten jeweils eine vorbestimmte Anzahl an physischen Kalibrierelementen aufweisen, welche derart räumlich angeordnet sind, dass ein auf der räumlichen Anordnung basierender Deskriptor unter der Transmissionsbildgebung projektiv invariant ist, und wobei dieser Deskriptor ein starker Deskriptor ist, durch welchen jeweils die Kalibrierobjekte der Vielzahl eindeutig identifizierbar sind; wobei:

das Zuordnen (322) für die physischen Kalibrierelemente der Kalibrierobjekte der Vielzahl an Kalibrierobjekten entsprechend ausgeführt wird; und das Zuordnen (322) aufweist:

- Identifizieren der einzelnen Kalibrierobjekte der Vielzahl an Kalibrierobjekten basierend auf dem starken Deskriptor.

3. Verfahren (300) gemäß Anspruch 1 oder 2, für welches das wenigstens eine Kalibrierobjekt (100) vier physische Kalibrierelemente aufweist (102, 104, 106, 108), die zumindest im Wesentlichen entlang einer ersten Geraden räumlich angeordnet sind, wobei:

der Deskriptor eine Anordnung der erkannten Kalibrierelemente zumindest im Wesentlichen entlang einer 2-D Geraden sowie ein Doppelverhältnis für die Abstände der erkannten Kalibrierelemente untereinander aufweist; beim Bestimmen der Untermengen (224) jene bestimmt werden, bei welchen die vier erkannten Kalibrierelemente der jeweiligen Untermenge zumindest im Wesentlichen entlang einer 2-D Geraden im 2-D Transmissionsbild angeordnet sind; und jeweils der Deskriptor je Untermenge des Weiteren mittels des Doppelverhältnisses für die Abstände der vier erkannten Kalibrierelemente untereinander berechnet wird.

4. Verfahren (300) gemäß einem der vorhergehenden Ansprüche, für welches das wenigstens eine Kalibrierobjekt (100) oder ein weiteres Kalibrierobjekt (110) sieben physische Kalibrierelemente aufweist, von denen eines auf einer ersten Geraden und auf einer zweiten Geraden, welche nicht parallel sind, liegt und von denen jeweils drei weitere zumindest im Wesentlichen entlang der ersten bzw. der zweiten Geraden räumlich angeordnet sind, wobei:

der Deskriptor eine Anordnung von vier erkannten Kalibrierelementen zumindest im Wesentlichen entlang einer ersten 2-D Geraden sowie ein erstes Doppelverhältnis für die Abstände dieser vier Kalibrierelemente untereinander und eine Anordnung von vier erkannten Kalibrierelementen entlang einer zweiten 2-D Geraden, welche die erste 2-D Gerade in einem dieser Kalibrierelemente schneidet, sowie ein zweites Doppelverhältnis für die Abstände dieser Kalibrierelemente untereinander aufweist; beim Bestimmen der Untermengen jene bestimmt werden, welche sieben erkannte Kalibrierelemente aufweisen, von denen vier entlang einer ersten 2-D Geraden und vier entlang einer zweiten 2-D Geraden im 2-D Transmissionsbild angeordnet sind; und jeweils der Deskriptor je Untermenge des Weiteren mittels des ersten Doppelverhältnisses für die Abstände der vier erkannten Kalibrierelemente entlang der ersten 2-D Geraden und mittels des Doppelverhältnisses für die Abstände der vier Kalibrierelemente entlang der zweiten 2-D Geraden berechnet wird.

5. Verfahren (300) gemäß einem der vorhergehenden Ansprüche, wobei das Zuordnen (322) des Weiteren aufweist:

- Festlegen (232) einer Reihenfolge der Kalibrierelemente (102, 104, 106, 108) für jede Untermenge mittels eines vorbestimmten Kriteriums für die Reihenfolge; und wobei der Deskriptor die Reihenfolge aufweist.

**6.** Verfahren (300) gemäß Anspruch 5, für welches das wenigstens eine Kalibrierobjekt (100) oder ein weiteres Kalibrierobjekt (110) wenigstens ein unterschiedliches Kalibrierelement (108; 114), das sich gegenüber den übrigen Kalibrierelementen (102, 104, 106; 112, 116, 118) bezüglich eines Unterscheidungskriteriums unterscheidet, aufweist, wobei das Zuordnen (322) des Weiteren aufweist:

- Bestimmen (228) jeweils des Unterscheidungskriteriums auf dem 2-D Transmissionsbild je erkannten Kalibrierelement (102, 104, 106, 108; 112, 114, 116, 118);
- Bestimmen (230) je Untermenge, ob und welche der erkannten Kalibrierelemente (102, 104, 106, 108; 112, 114, 116, 118) der jeweiligen Untermenge sich relativ zu den übrigen Kalibrierelementen (102, 104, 106; 112, 116, 118) durch ihr jeweiliges Unterscheidungskriterium unterscheiden; und wobei das vorbestimmte Kriterium für die Reihenfolge auf dem Unterscheidungskriterium basiert und/oder der Deskriptor eine Position des wenigstens einen unterschiedlichen Kalibrierelements (108; 114) innerhalb der Reihenfolge der Kalibrierelemente aufweist.

**7.** Verfahren (300) gemäß einem der vorhergehenden Ansprüche, für welches das 3-D Kalibrierphantom (10) ein weiteres Kalibrierobjekt (110) mit einer vorbestimmten Anzahl an physischen Kalibrierelementen (112, 114, 116, 118), welche entsprechend den physischen Kalibrierelementen (102, 104, 106, 108) des wenigstens einen Kalibrierobjekts (100) derart räumlich angeordnet und/oder ausgebildet sind, dass sich der Deskriptor bezüglich des weiteren Kalibrierobjekts (110) von dem Deskriptor bezüglich des wenigstens einen Kalibrierobjekts (100) unterscheidet, wobei:

das Zuordnen (322) für die physischen Kalibrierelemente (112, 114, 116, 118) des weiteren Kalibrierobjekts (110) entsprechend ausgeführt wird; und
sofern die vorbestimmte Anzahl an physischen Kalibrierelementen für das wenigstens eine Kalibrierobjekt und das weitere Kalibrierobjekt gleich sind, die erkannten Kalibrierelemente jener Untermenge entweder den physischen Kalibrierelementen des wenigstens einen Kalibrierobjekts oder den physischen Kalibrierelementen des weiteren Kalibrierobjekts abhängig davon zugeordnet werden, ob der berechnete Deskriptor für diese Untermenge von dem Deskriptor des wenigstens einen Kalibrierobjekts oder von dem Deskriptor des weiteren Kalibrierobjekts weniger abweicht.

**8.** Verfahren (300) gemäß einem der vorhergehenden Ansprüche, wobei das Zuordnen (322) des Weiteren aufweist:

- Berechnen (236) jeweils eines ersten Fehlerwertes je Untermenge, welcher die Abweichung zwischen dem für diese Untermenge berechneten Deskriptor und dem Deskriptor des wenigstens einen Kalibrierobjekts oder eines weiteren Kalibrierobjekts kennzeichnet;
und wobei die erkannten Kalibrierelemente einer der Untermengen den physischen Kalibrierelementen des wenigstens einen bzw. des weiteren Kalibrierobjekts abhängig davon zugeordnet werden (238), ob der jeweilige erste Fehlerwert geringer als ein erster Grenzwert ist.

**9.** Verfahren (300) gemäß einem der vorhergehenden Ansprüche, wobei das Zuordnen (322) mit unterschiedlich bestimmten Untermengen der erkannten Kalibrierelemente wiederholt ausgeführt wird.


### Claims

**1.** Method (300) for determining a geometry calibration for an imaging device (20), in which, in a transmission imaging, after passing through a 3D calibration phantom (10), radiation strikes a 2D detector (23) for creating a 2D transmission image of the 3D calibration phantom (10), wherein the 3D calibration phantom (10) has at least one calibration object (100) with a predetermined number of physical calibration elements (102, 104, 106, 108), which are spatially arranged in such a way that a descriptor based on the spatial arrangement is projectively invariant during the transmission imaging, wherein the physical calibration elements (102, 104, 106, 108; 112, 114, 116, 118) feature a material, which is opaque for the radiation and/or which scatters or absorbs the radiation, wherein the method features:

- provision (320) of the 2D transmission image, in which the at least one calibration object of the 3D calibration phantom is shown;
- assignment (322) of calibration elements recognised in the 2D transmission image to the physical calibration elements of the 3D calibration phantom;
- provision (324) of 3D arrangement data for the geometry calibration, which characterises the 3D arrangement of the physical calibration elements; and
- determination (328) of projection data of the geometry calibration, in particular of a projection matrix, for the 2D transmission image on the basis of the 3D arrangement data, of the calibration elements assigned to the physical calibration elements and recognised in the 2D transmission image and the 2D arrangement of the recognised calibration elements;

wherein the assignment (322) features:

- recognition (222) of the physical calibration elements (102, 104, 106, 108) shown in the 2D transmission image for determining calibration elements recognised in the 2D transmission image and their 2D arrangement;
- determination (224) of subsets of the recognised calibration elements, of which the number of calibration elements in each case corresponds to the predetermined number of physical calibration elements (102, 104, 106, 108) of the at least one calibration object (100);
- calculation (226) in each case of the descriptor relating to the calibration elements for each subset; and
- determination (238) of the assignment data, in that the recognised calibration elements of each subset are assigned to the physical calibration elements (102, 104, 106, 108) of the at least one calibration object (100), for which the computed descriptor and the descriptor relating to the physical calibration elements differ by the least from one another;

and wherein the method (300) furthermore features:

- provision (330) of one or more additional 2D transmission images relating to additional views of the 3D calibration phantom;
- respective assignment (332) of calibration elements recognised in the respective additional 2D transmission image to the physical calibration elements of the 3D calibration phantom; and
- provision (324) of the 3D arrangement data and determination (328) of the respective projection data of the geometry calibration for each 2D transmission image, in that the 3D arrangement data is modelled (334) so that with this 3D arrangement data the differences between the computed descriptors for the recognised calibration elements of the respective subsets of the respective 2D transmission images and the corresponding descriptors of the physical calibration elements are minimised.

2. Method (300) according to claim 1, for which the 3D calibration phantom (10) has a plurality of calibration objects, wherein the at least one calibration object (100) and a further calibration object (110, 120) are each part of the plurality of calibration objects, and wherein the calibration objects of the plurality of calibration objects each have a predetermined number of physical calibration elements, which are arranged spatially in such a way that a descriptor based on the spatial arrangement is projectively invariant during the transmission imaging, and wherein this descriptor is a strong descriptor, by way of which the calibration objects of the plurality are each able to be identified uniquely; wherein:
the assignment (322) is carried out accordingly for the physical calibration elements of the calibration objects of the plurality of calibration objects; and the assignment (322) features:

- Identification of the individual calibration objects of the plurality of calibration objects based on the strong descriptor.

3. Method (300) according to claim 1 or 2, for which the at least one calibration object (100) has four physical calibration elements (102, 104, 106, 108), which are at least essentially spatially arranged along a first straight line, wherein:

the descriptor features an arrangement of the recognised calibration elements at least essentially along a 2D straight line and also double ratio for the spacings between the recognised calibration elements;
during determination of the subsets (224) those subsets are determined, for which the four recognised calibration elements of the respective subset are arranged at least essentially along a 2D straight line in the 2D transmission image; and
the descriptor for each subset in each case is furthermore computed by means of the double ratio for the spacings between the four recognised calibration elements.

**4.** Method (300) according to one of the preceding claims, for which the at least one calibration object (100) or a further calibration object (110) has seven physical calibration elements, of which one lies on a first straight line and on a second straight line, which are not parallel, and of which three further elements in each case are at least essentially spatially arranged along the first or the second straight line, wherein:

the descriptor features an arrangement of four recognised calibration elements at least essentially along a first 2D straight line as well as a double ratio for the spacings between these four calibration elements and an arrangement of four recognised calibration elements along a second 2D straight line, which intersects with the first 2D straight line in one of these calibration elements, and also a second double ratio for the spacings between these calibration elements;

during determination of the subsets, those subsets are determined that have seven recognised calibration elements, of which four are arranged along a first 2D straight line and four along a second 2D straight line in the 2D transmission image; and

the descriptor for each subset in each case is furthermore computed by means of the first double ratio for the spacings of the four recognised calibration elements along the first 2D straight line and by means of the double ratio for the spacings of the four calibration elements along the second 2D straight line.

**5.** Method (300) according to one of the preceding claims, wherein the assignment (322) furthermore features:

- definition (232) of an order of the calibration elements (102, 104, 106, 108) for each subset by means of a predetermined criterion for the order;
and wherein the descriptor features the order.

**6.** Method (300) according to claim 5, for which the at least one calibration object (100) or a further calibration object (110) has at least one different calibration element (108; 114), which compared to the other calibration elements (102, 104, 106; 112, 116, 118) differs in relation to a distinction criterion, wherein the assignment (322) furthermore features:

- determination (228) in each case of the distinction criterion on the 2D transmission image for each recognised calibration element (102, 104, 106, 108; 112, 114, 116, 118);
- determination (230) for each subset, whether and which of the recognised calibration elements (102, 104, 106, 108; 112, 114, 116, 118) of the respective subset differs relative to the other calibration elements (102, 104, 106; 112, 116, 118) through its respective distinction criterion;
and wherein the predetermined criterion for the order is based on the distinction criterion and/or the descriptor features a position of the at least one different calibration element (108; 114) within the order of the calibration elements.

**7.** Method (300) according to one of the preceding claims, for which the 3D calibration phantom (10) has a further calibration object (110) with a predetermined number of physical calibration elements (112, 114, 116, 118), which are accordingly spatially arranged physical and/or embodied corresponding to calibration elements (102, 104, 106, 108) of the at least one calibration object (100) in such a way that the descriptor differs relating to the further calibration object (110) from the descriptor relating to the at least one calibration object (100), wherein:

the assignment (322) for the physical calibration elements (112, 114, 116, 118) of the further calibration object (110) is carried out accordingly; and

provided the predetermined number of physical calibration elements is the same for the at least one calibration object and the further calibration object, the recognised calibration elements of that subset are assigned either to the physical calibration elements of the at least one calibration object or the physical calibration elements of the further calibration object depending on whether the computed descriptor for these subsets differs less from the descriptor of the at least one calibration object or from the descriptor of the further calibration object.

**8.** Method (300) according to one of the preceding claims, wherein the assignment (322) furthermore features:

- calculation (236) in each case of a first error value for each subset, which characterises the difference between the descriptor computed for this subset and the descriptor of the at least one calibration object or of a further calibration object;

and wherein the recognised calibration elements of one of the subsets are assigned (238) to the physical calibration elements of the at least one or of the further calibration object depending on whether the respective first error value is less that a first limit value.

9. Method (300) according to one of the preceding claims, wherein the assignment (322) is carried out repeatedly with differently determined subsets of the recognised calibration elements.

## Revendications

1. Procédé (300) de détermination d'un étalonnage géométrique d'une installation (20) d'imagerie, dans laquelle, dans une imagerie de transmission, un rayonnement, après avoir traversé un fantôme (10) d'étalonnage en 3D, arrive sur un détecteur (23) en 2D pour l'établissement d'une image de transmission en 2D du fantôme (10) d'étalonnage en 3D, le fantôme (10) d'étalonnage en 3D ayant au moins un objet (100) d'étalonnage ayant un nombre déterminé à l'avance d'éléments (102, 104, 106, 108) physiques d'étalonnage, qui sont disposés dans l'espace de manière à ce qu'un descripteur reposant sur la disposition dans l'espace soit invariant en projection dans l'imagerie de transmission, les éléments (102, 104, 106, 108 ; 112, 114, 116, 118) physiques d'étalonnage ayant un matériau, qui est opaque au rayonnement et/ou qui absorbe ou diffuse le rayonnement, procédé dans lequel :

   - on se procure (320) l'image de transmission en 2D, dans laquelle le au moins un objet d'étalonnage du fantôme d'étalonnage en 3D est représenté ;
   - on attribue (322) des éléments d'étalonnage reconnus dans l'image de transmission en 2D aux éléments physiques d'étalonnage du fantôme d'étalonnage en 3D ;
   - on se procure (324) des données de disposition en 3D pour l'étalonnage géométrique, qui caractérisent la disposition en 3D des éléments physiques d'étalonnage ;
   - on détermine (328) des données de projection de l'étalonnage géométrique, notamment une matrice de projection, pour l'image de transmission en 2D sur la base des données de disposition en 3D, des éléments d'étalonnage attribués aux éléments physiques d'étalonnage et reconnus dans l'image de transmission en 2D et de la disposition en 2D des éléments d'étalonnage reconnus ;

   dans lequel l'attribution (322) comporte :

   - la reconnaissance (222) des éléments (102, 104, 106, 108) physiques d'étalonnage représentés dans l'image de transmission en 2D pour la détermination d'éléments d'étalonnage reconnus dans l'image de transmission en 2D et de leur disposition en 2D ;
   - la détermination (224) de sous-ensembles des éléments d'étalonnage reconnus, dont le nombre d'éléments d'étalonnage correspond respectivement au nombre déterminé à l'avance d'éléments (102, 104, 106, 108) physiques d'étalonnage du au moins un objet (100) d'étalonnage ;
   - le calcul (226) respectivement du descripteur se rapportant aux éléments d'étalonnage par sous-ensemble ;
   - la détermination (238) des données d'attribution, en attribuant des éléments d'étalonnage reconnus de chaque sous-ensemble aux éléments (102, 104, 106, 108) physiques d'étalonnage du au moins un objet (100) d'étalonnage pour lesquels le descripteur calculé et le descripteur se rapportant aux éléments physiques d'étalonnage s'écartent le moins l'un de l'autre ;

   et dans lequel en outre, dans le procédé (300) :

   - on se procure (330) une ou plusieurs images de transmission en 2D supplémentaires se rapportant à des vues supplémentaires du fantôme d'étalonnage en 3D ;
   - on affecte (332) respectivement des éléments d'étalonnage reconnus dans l'image de transmission en 2D supplémentaire respective aux éléments physiques d'étalonnage du fantôme d'étalonnage en 3D ;
   - on se procure (324) les données de disposition en 3D et on détermine (328) les données de projection respectives de l'étalonnage géométrique par image de transmission en 2D, en modélisant (334) les données de disposition en 3D de manière à minimiser, par ces données de disposition en 3D, les écarts entre les descripteurs calculés pour les éléments d'étalonnage reconnus des sous-ensembles respectifs des images de transmission en 2D respectives, aux descripteurs correspondants des éléments physiques d'étalonnage.

2. Procédé (300) suivant la revendication 1, pour lequel le fantôme (10) d'étalonnage en 3D a une pluralité d'objets d'étalonnage, dans lequel le au moins un objet (100) d'étalonnage et un autre objet (110, 120) d'étalonnage sont respectivement une partie de la pluralité d'objets d'étalonnage et dans lequel les objets d'étalonnage de la pluralité d'objets d'étalonnage ont respectivement un nombre déterminé à l'avance d'éléments physiques d'étalonnage, sont disposés dans l'espace de manière à ce qu'un descripteur reposant sur la disposition dans l'espace soit invariant en projection dans l'imagerie en transmission et dans lequel ce descripteur est un descripteur fort, par lequel respectivement les objets de calibrage de la pluralité peuvent être identifiés d'une manière univoque ; dans lequel : on effectue l'affectation (322) des éléments physiques d'étalonnage et des objets d'étalonnage à la pluralité d'objets d'étalonnage de manière correspondante ; et l'affectation (322) comporte :

- l'identification des divers objets d'étalonnage de la pluralité d'objets d'étalonnage sur la base du descripteur fort.

3. Procédé (300) suivant la revendication 1 ou 2, pour lequel le au moins un objet (100) d'étalonnage a quatre éléments (102, 104, 106, 108) physiques d'étalonnage, qui sont disposés dans l'espace, au moins pour l'essentiel, suivant une première droite, dans lequel :

le descripteur a une disposition des éléments d'étalonnage reconnus au moins sensiblement suivant une droite en 2D, ainsi qu'un rapport anharmonique des distances entre les éléments d'étalonnage reconnus ; lors de la détermination des sous-ensembles (224), on détermine ceux pour lesquels les quatre éléments d'étalonnage reconnus du sous-ensemble respectif sont disposés au moins sensiblement suivant une droite en 2D dans l'image de transmission en 2D ;

- on calcule respectivement le descripteur de chaque sous-ensemble en outre au moyen du rapport anharmonique des distances des quatre éléments d'étalonnage reconnus entre eux.

4. Procédé (300) suivant l'une des revendications précédentes, pour lequel le au moins un objet (100) d'étalonnage ou un autre objet (110) d'étalonnage a sept éléments physiques d'étalonnage, dont l'un est sur une première droite et sur une deuxième droite, qui ne sont pas parallèles, et dont respectivement trois autres sont disposés au moins pour l'essentiel dans l'espace suivant la première ou la deuxième droite, dans lequel :

le descripteur a une disposition de quatre éléments d'étalonnage reconnus au moins pour l'essentiel suivant une première droite en 2D, ainsi qu'un premier rapport anharmonique des distances de ces quatre éléments d'étalonnage entre eux et une disposition de quatre éléments d'étalonnage reconnus suivant une deuxième droite en 2D, qui coupe la première droite en 2D dans l'un de ces éléments d'étalonnage, ainsi qu'un deuxième rapport anharmonique des distances de ces éléments d'étalonnage entre eux ; lors de la détermination des sous-ensembles, on détermine ceux qui ont sept éléments d'étalonnage reconnus, dont quatre sont disposés selon une première droite en 2D et quatre suivant une deuxième droite en 2d dans l'image de transmission en 2D ; on calcule respectivement le descripteur par sous-ensemble en outre au moyen du premier rapport anharmonique des distances des quatre éléments d'étalonnage reconnus, suivant la première droite en 2D et au moyen du rapport anharmonique des distances des quatre éléments d'étalonnage suivant la deuxième droite en 2d ;

5. Procédé (300) suivant l'une des revendications précédentes, dans lequel l'attribution (322) comporte en outre :

- la fixation (232) d'une suite des éléments (102, 104, 106, 108) d'étalonnage pour chaque sous-ensemble au moyen d'un critère défini à l'avance pour la suite ; et dans lequel le descripteur comporte la suite.

6. Procédé (300) suivant la revendication 5, pour lequel le au moins un objet (100) d'étalonnage ou un autre objet (110) d'étalonnage a au moins un élément (108 ; 114) d'étalonnage différent, qui se distingue des autres éléments (102, 104, 106 ; 112, 116, 118) d'étalonnage par un critère de distinction, l'attribution (322) comportant en outre :

- la détermination (228) respectivement du critère de distinction sur l'image de transmission en 2D pour chaque élément (102, 104, 106, 108 ; 112, 114, 116, 118) d'étalonnage reconnu ; - la détermination (230) pour chaque sous-ensemble du point de savoir si ceux des éléments (102, 104, 106, 108 ; 112, 114, 116, 118) d'étalonnage reconnus du sous-ensemble respectif se distinguent par rapport aux autres éléments (102, 104, 106 ; 112, 116, 118) d'étalonnage par leurs critères de distinction respectif ; et dans lequel le critère défini à l'avance pour la suite repose sur le critère de distinction et/ou le descripteur a une position du au moins un élément (108 ; 114) d'étalonnage différente dans la suite des éléments d'étalonnage.

7. Procédé (300) suivant l'une des revendications précédentes, pour lequel le fantôme (10) d'étalonnage en 3D a un autre objet (110) d'étalonnage ayant un nombre défini à l'avance d'éléments (112, 114, 116, 118) physiques d'étalonnage qui sont disposés et/ou constitués dans l'espace conformément aux éléments (102, 104, 106, 108) physiques d'étalonnage du au moins un objet (100) d'étalonnage de manière à ce que le descripteur se rapporte à l'autre objet (110) d'étalonnage se distingue du descripteur se rapportant au au moins un objet (100) d'étalonnage, dans lequel :

on effectue de manière correspondante l'attribution (322) pour les éléments (112, 114, 116, 118) physiques d'étalonnage de l'autre objet (110) d'étalonnage ;

dans la mesure où le nombre défini à l'avance d'éléments physiques d'étalonnage pour le au moins un objet d'étalonnage et l'autre objet étalonnage sont égaux, on attribue les éléments d'étalonnage reconnus de chaque sous-ensemble soit aux éléments physiques d'étalonnage du au moins un objet d'étalonnage soit aux éléments physiques d'étalonnage de l'autre objet d'étalonnage selon que le descripteur calculé pour ce sous-ensemble s'écarte moins du descripteur du au moins un objet d'étalonnage ou du descripteur de l'autre objet d'étalonnage.

8. Procédé (300) suivant l'une des revendications précédentes, dans lequel l'attribution (322) comporte en outre :

- le calcul (236) respectivement d'une première valeur d'erreur par sous-ensemble, qui caractérise l'écart entre le descripteur calculé pour ce sous-ensemble et le descripteur du au moins un objet d'étalonnage ou d'un autre objet d'étalonnage ;

et dans lequel on attribue (238) les éléments d'étalonnage reconnus de l'un des sous-ensembles aux éléments physiques d'étalonnage du au moins un ou de l'autre objet d'étalonnage selon que la première valeur d'erreur respective est plus petite qu'une première valeur limite.

9. Procédé (300) suivant l'une des revendications précédentes, dans lequel on effectue l'attribution (322) de manière répétée avec des sous-ensembles définis de manière différente des éléments d'étalonnage reconnus.

FIG 1

# FIG 2

132    134    136                                                    138

140
141
142
143
144
145
146
147
148
149
150
151
152
153
154
155
156
157
158
159
160

FIG 3

## FIG 4

200

FIG 5

FIG 6

FIG 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0187136 A2
- US 20170074808 A1

**In der Beschreibung aufgeführte Nicht-Patentliteratur**

- **N. K. STROBEL et al.** Improving 3-D Image Quality of X-Ray C-Arm Imaging Systems by Using Properply Designed Pose Determination Systems for Calibrating the Projection Geometry. *Proceedings of SPIE*